(19) Europäisches Patentamt
European Patent Office
Office européen des brevets

(11) EP 0 552 765 B1

(12) EUROPÄISCHE PATENTSCHRIFT

(45) Veröffentlichungstag und Bekanntmachung des Hinweises auf die Patenterteilung:
**14.08.1996 Patentblatt 1996/33**

(51) Int. Cl.[6]: **C07D 403/04**, A61K 31/41, C07D 413/04, C07D 417/04, C07D 403/14, C07D 413/14, C07D 417/14

(21) Anmeldenummer: **93100890.8**

(22) Anmeldetag: **21.01.1993**

(54) **Benzimidazole, diese Verbindungen enthaltende Arzneimittel und Verfahren zu ihrer Herstellung**

Benzimidazoles, pharmaceutical compositions containing them and process for their preparation

Benzimidazoles, médicaments les contenant et procédé pour leur préparation

(84) Benannte Vertragsstaaten:
**AT BE CH DE DK ES FR GB GR IE IT LI LU NL PT SE**

(30) Priorität: **22.01.1992 DE 4201554**
**17.06.1992 DE 4219782**
**04.08.1992 DE 4225756**

(43) Veröffentlichungstag der Anmeldung:
**28.07.1993 Patentblatt 1993/30**

(73) Patentinhaber: **Dr. Karl Thomae GmbH**
**D-88397 Biberach (DE)**

(72) Erfinder:
- **Hauel, Norbert, Dr. Dipl.-Chem.**
  **W-7957 Schemmerhofen (DE)**
- **Narr, Berthold, Dr. Dipl.-Chem.**
  **W-7950 Biberach 1 (DE)**
- **Ries, Uwe, Dr. Dipl.-Chem.**
  **W-7950 Biberach 1 (DE)**
- **van Meel, Jacques, Dr.**
  **W-7951 Mittelbiberach (DE)**
- **Wienen, Wolfgang, Dr. Dipl.-Biologe**
  **W-7951 Apfingen (DE)**
- **Entzeroth, Michael, Dr. Dipl.-Chem.**
  **W-7951 Warthausen (DE)**

(56) Entgegenhaltungen:
**EP-A- 0 392 317**
**EP-A- 0 400 835**
**EP-A- 0 468 470**
**EP-A- 0 502 314**

Bemerkungen:
Die Akte enthält technische Angaben, die nach dem Eingang der Anmeldung eingereicht wurden und die nicht in dieser Patentschrift enthalten sind.

EP 0 552 765 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.0/3.4

## Beschreibung

In der EP-A-0 392 317 werden bereits Benzimidazole, welche wertvolle Angiotensin-Antagonisten darstellen, beschrieben.

In der EP-A-0,502,314, welche nur teilweise eine Vorveröffentlichung darstellt, werden bereits Verbindungen der allgemeinen Formel I beschrieben, in denen $R_2$ eine Imidazol-4-yl-Gruppe darstellt, welche durch eine oder mehrere unsubstituierte Alkyl-, Phenylalkyl- oder Cycloalkylgruppen substituiert sein kann.

Es wurde nun gefunden, daß die neuen Benzimidazole der allgemeinen Formel

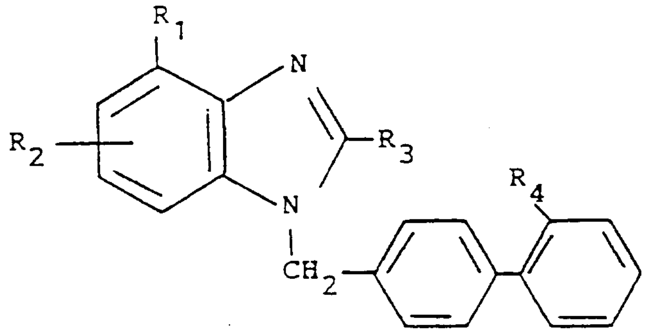

, (I)

in der

$R_1$ eine Alkylgruppe mit 1 bis 3 Kohlenstoffatomen, ein Wasserstoff-, Fluor-, Chlor- oder Bromatom,

$R_2$ eine in 2-Stellung durch eine Phenylgruppe substituierte Oxazol-4-yl- oder Thiazol-4-yl-Gruppe oder eine gegebenenfalls in 2-Stellung durch eine Alkylgruppe mit 1 bis 6 Kohlenstoffatomen oder durch eine Phenylgruppe substituierte Imidazol-4-yl-Gruppe, wobei die Imidazol-4-yl-Gruppe in 1-Stellung durch eine Alkylgruppe mit 1 bis 7 Kohlenstoffatomen, die in 1-, 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Alkoxy-, Alkoxyalkoxy-, Dialkylamino-, Pyrrolidino-, Piperidino-, Hexamethylenimino-, Morpholino-, Thiomorpholino-, 1-Oxido-thiomorpholino- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine durch Fluor- oder Chloratome, durch Trifluormethyl-, Methyl- oder Methoxygruppen mono- oder disubstituierte Phenylgruppe substituiert ist, oder durch eine durch zwei Phenylgruppen substituierte Alkylgruppe substituiert ist, wobei, soweit nichts anderes erwähnt wurde, die vorstehend erwähnten Alkyl- und Alkoxyteile jeweils 1 bis 3 Kohlenstoffatome enthalten können,

$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und

$R_4$ eine in vivo in eine Carboxygruppe überführbare Gruppe, eine Carboxy-, Cyano-, 1H-Tetrazolyl-, 1-Triphenylmethyltetrazolyl- oder 2-Triphenylmethyl-tetrazolylgruppe bedeuten,

sowie die Verbindungen

a) 4'-[(2-n-Propyl-4-methyl-6-(1-(2-phenylethyl)-imidazol-4-yl)-benzimidazol-1-yl)-methyl]-biphenyl-2-carbonsäure,

b) 4'-[(2-n-Propyl-4-methyl-6-(2-methyl-oxazol-4-yl)-benzimidazol-1-yl)-methyl]-biphenyl-2-carbonsäure,

c) 4'-[(2-n-Propyl-4-methyl-6-(2-methyl-oxazol-4-yl)-benzimidazol-1-yl)-methyl]-2-(1H-tetrazol-5-yl)-biphenyl,

d) 4'-[(2-Ethoxy-6-(1-isopropyl-imidazol-4-yl)-benzimidazol-1-yl)-methyl]-2-(1H-tetrazol-5-yl)-biphenyl,

e) 4'-[(2-Ethoxy-6-(1-isopropyl-imidazol-4-yl)-benzimidazol-1-yl)-methyl]-biphenyl-2-carbonsäure,

f) 4'-[(2-Ethoxy-5-(1-isopropyl-imidazol-4-yl)-benzimidazol-1-yl)-methyl]-2-(1H-tetrazol-5-yl)-biphenyl,

g) 4'-[(2-n-Propyl-4-methyl-6-(1-cycloheptyl-imidazol-4-yl)-benzimidazol-1-yl)-methyl]-biphenyl-2-carbonsäure,

h) 4'-[(2-n-Propyl-4-methyl-6-(1-cycloheptyl-imidazol-4-yl)-benzimidazol-1-yl)-methyl]-2-(1H-tetrazol-5-yl)-biphenyl,

i) 4'-[(2-n-Propyl-4-methyl-6-(1-(1-n-propyl-n-butyl)-imidazol-4-yl)-benzimidazol-1-yl)-methyl]-2-(1H-tetrazol-5-yl)-biphenyl,

j) 4'-[(2-n-Propyl-4-methyl-6-(1,2-dimethyl-imidazol-4-yl)-benzimidazol-1-yl)-methyl]-biphenyl-2-carbonsäure,

k) 4'-[(2-n-Propyl-4-methyl-6-(1,2-dimethyl-imidazol-4-yl)-benzimidazol-1-yl)-methyl]-2-(1H-tetrazol-5-yl)-biphenyl,

l) 4'-[(2-n-Propyl-4-methyl-6-(2-methyl-thiazol-4-yl)-benzimidazol-1-yl)-methyl]-biphenyl-2-carbonsäure und

m) 4'-[(2-n-Propyl-4-methyl-6-(2-methyl-thiazol-4-yl)-benzimidazol-1-yl)-methyl]-2-(1H-tetrazol-5-yl)-biphenyl,

und deren Salze, insbesondere für die pharmazeutische Anwendung deren physiologisch verträglichen Salze mit anorganischen oder organischen Säuren oder Basen, ebenfalls Angiotensin-Antagonisten, insbesondere Angiotensin-II-Antagonisten, darstellen.

Unter dem vorstehend erwähnten Begriff "eine Gruppe, die in vivo in eine Carboxygruppe übergeführt wird," sind beispielsweise deren Ester der Formeln

- CO - OR',

- CO - O - (HCR") - O - CO - R'" und

- CO - O - (HCR") - O - CO - OR'"

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R" ein Wasserstoffatom oder eine Methylgruppe und
R'" eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen.

Die neuen Verbindungen der obigen Formel I weisen wertvolle Eigenschaften auf. So weisen die Verbindungen der Formel I, in der $R_4$ eine in vivo in eine Carboxygruppe überführbare Gruppe, eine Carboxy- oder 1H-Tetrazolylgruppe bedeutet, insbesondere wertvolle pharmakologische Eigenschaften auf, da diese Angiotensin-Antagonisten, insbesondere Angiotensin-II-Antagonisten, darstellen. Die übrigen Verbindungen der allgemeinen Formel I stellen wertvolle Zwischenprodukte zur Herstellung der vorstehend erwähnten Verbindung dar.

Gegenstand der vorliegenden Erfindung sind somit die neuen vorstehend erwähnten Benzimidazole, wobei die entsprechenden Alkoxycarbonylverbindungen gleichzeitig auch wertvolle Zwischenprodukte darstellen, deren Verwendung und deren Herstellung.

Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind, und deren Herstellung.

Für die bei der Definition der Reste $R_1$ bis $R_4$ eingangs erwähnten Bedeutungen kommt beispielsweise für
$R_1$ die Bedeutung des Wasserstoff-, Fluor- oder Chloratoms, die der Methyl-, Ethyl-, n-Propyl- oder Isopropylgruppe,
$R_2$ die der 2-Phenyl-oxazol-4-yl-, 2-Phenyl-thiazol-4-yl-, 1-Cyclopropylmethyl-imidazol-4-yl-, 1-Cyclobutylmethyl-imidazol-4-yl-, 1-Cyclopentylmethyl-imidazol-4-yl-, 1-Cyclohexylmethyl-imidazol-4-yl-, 1-Cycloheptylmethyl-imidazol-4-yl-, 1-(2-Cyclo- propylethyl)-imidazol-4-yl-, 1-(2-Cyclobutylethyl)-imidazol-4-yl-, 1-(2-Cyclopentylethyl)-imidazol-4-yl-, 1-(2-Cyclo- hexylethyl)-imidazol-4-yl-, 1-(2-Cycloheptylethyl)-imidazol-4-yl-, 1-(3-Cyclopropylpropyl)-imidazol-4-yl-, 1-(3-Cyclobu- tylpropyl)-imidazol-4-yl-, 1-(3-Cyclopentylpropyl)-imidazol-4-yl-, 1-(3-Cyclohexylpropyl)-imidazol-4-yl-, 1-(3-Cycloheptylpropyl)-imidazol-4-yl-, 1-(2,2,2-Trifluorethyl)-imidazol-4-yl-, 1-(3,3,3-Trifluorpropyl)-imidazol-4-yl-, 1-(4-Fluor-benzyl)-imidazol-4-yl-, 1-(4-Chlor-benzyl)-imidazol-4-yl-, 1-(3-Chlor-benzyl)-imidazol-4-yl-, 1-(4-Trifluormethyl-benzyl)-imidazol-4-yl-, 1-(3-Methyl-benzyl)-imidazol-4-yl-, 1-(4-Methyl-benzyl)-imidazol-4-yl-, 1-(3-Methoxy-benzyl)-imidazol-4-yl-, 1-(4-Methoxy-benzyl)-imidazol-4-yl-, 1-(3,4-Dimethoxy-benzyl)-imidazol-4-yl-, 1-(3,5-Dimethoxy-benzyl)-imidazol-4-yl-, 1-Cyclopropylmethyl-2-methyl-imidazol-4-yl-, 1-Cyclobutylmethyl-2-methyl-imidazol-4-yl-, 1-Cyclopentylmethyl-2-methyl-imidazol-4-yl-, 1-Cyclohexylmethyl-2-methyl-imidazol-4-yl-, 1-Cycloheptylmethyl-2-methyl-imidazol-4-yl-, 1-(2-Cyclopropylethyl)-2-methyl-imidazol-4-yl-, 1-(2-Cyclobutylethyl)-2-methyl-imidazol-4-yl-, 1-(2-Cyclopentylethyl)-2-methyl-imidazol-4-yl-, 1-(2-Cyclohexylethyl)-2-methyl-imidazol-4-yl-, 1-(2-Cycloheptylethyl)-2-methyl-imidazol-4-yl-, 1-(3-Cyclopropylpropyl)-2-methyl-imidazol-4-yl-, 1-(3-Cyclobutylpropyl)-2-methyl-imidazol-4-yl-, 1-(3-Cyclopentylpropyl)-2-methyl-imidazol-4-yl-, 1-(3-Cyclohexylpropyl)-2-methyl-imidazol-4-yl-, 1-(3-Cycloheptylpropyl)-2-methyl-imidazol-4-yl-, 1-(2,2,2-Trifluorethyl)-2-methyl-imidazol-4-yl-, 1-(3,3,3-Trifluorpropyl)-2-methyl-imidazol-4-yl-, 1-(4-Fluor-benzyl)-2-methyl-imidazol-4-yl-, 1-(4-Chlor-benzyl)-2-methyl-imidazol-4-yl-, 1-(3-Chlor-benzyl)-2-methyl-imidazol-4-yl-, 1-(4-Trifluormethyl-benzyl)-2-methyl-imidazol-4-yl-, 1-(3-Methyl-benzyl)-2-methyl-imidazol-4-yl-, 1-(4-Methyl-benzyl)-2-methyl-imidazol-4-yl-, 1-(3-Methoxy-benzyl)-2-methyl-imidazol-4-yl-, 1-(4-Methoxy-benzyl)-2-methyl-imidazol-4-yl-, 1-(3,4-Dimethoxy-benzyl)-2-methyl-imidazol-4-yl-, 1-(3,5-Dimethoxy-benzyl)-2-methyl-imidazol-4-yl-, 1-Carboxymethyl-imidazol-4-yl-, 1-(2-Carboxyethyl)-imidazol-4-yl-, 1-(3-Carboxypropyl)-imidazol-4-yl-, 1-(4-Carboxybutyl)-imidazol-4-yl-, 1-(5-Carboxypentyl)-imidazol-4-yl-, 1-(6-Carboxyhexyl)-imidazol-4-yl-, 1-(7-Carboxyheptyl)-imidazol-4-yl-, 1-Methoxycarbonylmethyl-imidazol-4-yl-, 1-(2-Methoxycarbonylethyl)-imidazol-4-yl-, 1-(3-Methoxycarbonylpropyl)-imidazol-4-yl-, 1-(4-Methoxycarbonylbutyl)-imidazol-4-yl-, 1-(5-Methoxycarbonylpentyl)-imidazol-4-yl-, 1-(6-Methoxycarbonylhexyl)-imidazol-4-yl-, 1-(7-Methoxycarbonylheptyl)-imidazol-4-yl-, 1-Ethoxycarbonylmethyl-imidazol-4-yl-, 1-(2-Ethoxycarbonylethyl)-imidazol-4-yl-, 1-(3-Ethoxycarbonylpropyl)-imidazol-4-yl-, 1-(4-Ethoxycarbonylbutyl)-imidazol-4-yl-, 1-(5-Ethoxycarbonylpentyl)-imidazol-4-yl-, 1-(6-Ethoxycarbonylhexyl)-imidazol-4-yl-, 1-(7-Ethoxycarbonylheptyl)-imidazol-4-yl-, 1-n-Propoxycarbo- nylmethyl-imidazol-4-yl-, 1-(2-n-Propoxycarbonylethyl)-imidazol-4-yl-, 1-(3-n-Propoxycarbonylpropyl)-imidazol-4-yl-, 1-(4-n-Propoxycarbonylbutyl)-imidazol-4-yl-, 1-(5-n-Propoxycarbonylpentyl)-imidazol-4-yl-, 1-(6-n-Propoxycarbonylhexyl)-imidazol-4-yl-, 1-(7-n-Propoxycarbonylheptyl)-imidazol-4-yl-, 1-Isopropoxycarbonylmethyl-imidazol-4-yl-, 1-(2-Isopropoxy- carbonylethyl)-imidazol-4-yl-, 1-(3-Isopropoxycarbonylpropyl)-imidazol-4-yl-, 1-(4-Isopropoxycarbonylbutyl)-imidazol-4-yl-, 1-(5-Isopropoxycarbonylpentyl)-imidazol-4-yl-, 1-(6-Isopropoxycarbonylhexyl)-imidazol-4-yl-, 1-(7-Isopropoxycarbonylheptyl)-imidazol-4-yl-, 1-Aminocarbonylmethyl-imidazol-4-yl-, 1-(2-Aminocarbonylethyl)-imidazol-4-yl-, 1-(3-Amino- carbonylpropyl)-imidazol-4-yl-, 1-(4-Aminocarbonylbutyl)-imidazol-4-yl-, 1-(5-Aminocarbonylpentyl)-imidazol-4-yl-, 1-(6-Aminocarbonylhexyl)-imidazol-4-yl-, 1-(7-Aminocarbonylheptyl)-imidazol-4-yl-, 1-Methylaminocarbonylmethyl-imidazol-4-yl-, 1-(2-Methylaminocarbonylethyl)-imidazol-4-yl-, 1-(3-Methylaminocarbonylpropyl)-imidazol-4-yl-, 1-(4-Methylaminocarbonylbutyl)-imidazol-4-yl-, 1-(5-Methylaminocarbonylpentyl)-imidazol-4-yl-, 1-(6-Methylaminocarbonylhexyl)-imidazol-4-yl-, 1-(7-Methylaminocarbonylheptyl)-imidazol-4-yl-, 1-Ethylaminocarbonylmethyl-imidazol-4-yl-, 1-(2-Ethylaminocarbonylethyl)-imidazol-4-yl-, 1-(3-Ethylaminocarbonylpropyl)-imidazol-4-yl-, 1-(4-Ethylaminocarbonylbutyl)-imidazol-4-yl-, 1-(5-Ethylaminocarbonylpentyl)-imidazol-4-yl-, 1-(6-Ethylaminocarbonylhexyl)-imidazol-4-yl-, 1-(7-Ethylaminocarbonylheptyl)-imidazol-4-yl-, 1-n-Propylaminocarbonylmethyl-imidazol-4-yl-, 1-(2-n-Propylaminocarbonylethyl)-imidazol-4-yl-, 1-(3-n-Propylaminocarbonylpropyl)-imidazol-4-yl-, 1-(4-n-Propylaminocarbonylbutyl)-imidazol-4-yl-, 1-(5-n-Propylaminocarbonylpentyl)-imidazol-4-yl-, 1-(6-n-Propylaminocarbonylhexyl)-imidazol-4-yl-, 1-(7-n-Propylaminocarbonylheptyl)-imidazol-4-yl-, 1-Isopropylaminocarbonylmethyl-imidazol-4-yl-, 1-(2-Isopropylaminocarbonylethyl)-imidazol-4-yl-, 1-(3-Isopropylaminocarbonylpropyl)-imidazol-4-yl-, 1-(4-Isopropylaminocarbonylbutyl)-imidazol-4-yl-, 1-(5-Isopropylaminocarbonylpentyl)-imidazol-4-yl-, 1-(6-Isopropylaminocarbonylhexyl)-imidazol-4-yl-, 1-(7-Isopropylaminocarbonylheptyl)-imidazol-4-yl-, 1-Dimethylaminocarbonylmethyl-imidazol-4-yl-, 1-(2-Dimethylaminocarbonylethyl)-imidazol-4-yl-, 1-(3-Dimethylaminocarbonylpropyl)-imidazol-4-yl-, 1-(4-Dimethylaminocarbonylbutyl)-imidazol-4-yl-, 1-(5-Dimethylaminocarbonylpentyl)-imidazol-4-yl-, 1-(6-Dimethylaminocarbonylhexyl)-imidazol-4-yl-, 1-(7-Dimethylaminocarbonylheptyl)-imidazol-4-yl-, 1-Diethylaminocarbonylmethyl-imidazol-4-yl-, 1-(2-Diethylaminocarbonylethyl)-imidazol-4-yl-, 1-(3-Diethylaminocarbonylpropyl)-imidazol-4-yl-, 1-(4-Diethylaminocarbonylbutyl)-imidazol-4-yl-, 1-(5-Diethylaminocarbonylpentyl)-imidazol-4-yl-, 1-(6-Diethylaminocarbonylhexyl)-imidazol-4-yl-, 1-(7-Diethylaminocarbonylheptyl)-imidazol-4-yl-, 1-Di-n-propylaminocarbonylmethyl-imidazol-4-yl-, 1-(2-Di-n-propylaminocarbonylethyl)-imidazol-4-yl-, 1-(3-Di-n-propylaminocarbonylpropyl)-imidazol-4-yl-, 1-(4-Di-n-propylaminocarbonylbutyl)-imidazol-4-yl-, 1-(5-Di-n-propylaminocarbonylpentyl)-imidazol-4-yl-, 1-(6-Di-n-propylaminocarbonylhexyl)-imidazol-4-yl-, 1-(7-Di-n-propylaminocarbonylheptyl)-imidazol-4-yl-, 1-Diisopropylaminocarbonylmethyl-imidazol-4-yl-, 1-(2-Diisopropylaminocarbonylethyl)-imidazol-4-yl-, 1-(3-Diisopropylaminocarbonylpropyl)-imidazol-4-yl-, 1-(4-Diisopropylaminocarbonylbutyl)-imidazol-4-yl-, 1-(5-Diisopropylaminocarbonylpentyl)-imidazol-4-yl-, 1-(6-Diisopropylaminocarbonylhexyl)-imidazol-4-yl-, 1-(7-Diisopropylaminocarbonylheptyl)-imidazol-4-yl-, 1-Morpholinocarbonylmethyl-imidazol-4-yl-, 1-(2-Morpholinocarbonylethyl)-imidazol-4-yl-, 1-(3-Morpholinocarbonylpropyl)-imidazol-4-yl-, 1-(4-Morpholinocarbonylbutyl)-imidazol-4-yl-, 1-(5-Morpholinocarbonylpentyl)-imidazol-4-yl-, 1-(6-Morpholinocarbonylhexyl)-imidazol-4-yl-, 1-(7-Morpholinocarbonylheptyl)-imidazol-4-yl-, 1-Thiomorpholinocarbonylmethyl-imidazol-4-yl-, 1-(2-Thiomorpholinocarbonylethyl)-imidazol-4-yl-, 1-(3-Thiomorpholinocarbonylpropyl)-imidazol-4-yl-, 1-(4-Thiomorpholinocarbonylbutyl)-imidazol-4-yl-, 1-(5-Thiomorpholinocarbonylpentyl)-imidazol-4-yl-, 1-(6-Thiomorpholinocarbonylhexyl)-imidazol-4-yl-, 1-(7-Thiomorpholinocarbonylheptyl)-imidazol-4-yl-, 1-Oxidothiomorpholinocarbonylmethyl-imidazol-4-yl-, 1-(2-Oxidothiomorpholinocarbonylethyl)-imidazol-4-yl-, 1-(3-Oxidothiomorpholinocarbonylpropyl)-imidazol-4-yl-, 1-(4-Oxidothiomorpholinocarbonylbutyl)-imidazol-4-yl-, 1-(5-Oxidothiomorpholinocarbonylpentyl)-imidazol-4-yl-, 1-(6-Oxidothiomorpholinocarbonylhexyl)-imidazol-4-yl-, 1-(7-Oxidothiomorpholinocarbonylheptyl)-imidazol-4-yl-, 1-Carboxymethyl-2-methyl-imidazol-4-yl-, 1-(2-Carboxyethyl)-2-methyl-imidazol-4-yl-, 1-(3-Carboxypropyl)-2-methyl-imidazol-4-yl-, 1-(4-Carboxybutyl)-2-methyl-imidazol-4-yl-, 1-(5-Carboxypentyl)-2-methyl-imidazol-4-yl-, 1-(6-Carboxyhexyl)-2-methyl-imidazol-4-yl-, 1-(7-Carboxyheptyl)-2-methyl-imidazol-4-yl-, 1-Methoxycarbonylmethyl-2-methyl-imidazol-4-yl-, 1-(2-Methoxycarbonylethyl)-2-methyl-imidazol-4-yl-, 1-(3-Methoxycarbonylpropyl)-2-methyl-imidazol-4-yl-, 1-(4-Methoxycarbonylbutyl)-2-methyl-imidazol-4-yl-, 1-(5-Methoxycarbonylpentyl)-2-methyl-imidazol-4-yl-, 1-(6-Methoxycarbonylhexyl)-2-methyl-imidazol-4-yl-, 1-(7-Methoxycarbonylheptyl)-2-methyl-imidazol-4-yl-, 1-Ethoxycarbonylmethyl-2-methyl-imidazol-4-yl-, 1-(2-Ethoxycarbonylethyl)-2-methyl-imidazol-4-yl-, 1-(3-Ethoxycarbonylpropyl)-2-methyl-imidazol-4-yl-, 1-(4-Ethoxycarbonylbutyl)-2-methyl-imidazol-4-yl-, 1-(5-Ethoxycarbonylpentyl)-2-methyl-imidazol-4-yl-, 1-(6-Ethoxycarbonylhexyl)-2-methyl-imidazol-4-yl-, 1-(7-Ethoxycarbonylheptyl)-2-methyl-imidazol-4-yl-, 1-n-Propoxycarbonylmethyl-2-methyl-imidazol-4-yl-, 1-(2-n-Propoxycarbonylethyl)-2-methyl-imidazol-4-yl-, 1-(3-n-Propoxycarbonylpropyl)-2-methyl-imidazol-4-yl-, 1-(4-n-Propoxycarbonylbutyl)-2-methyl-imidazol-4-yl-, 1-(5-n-Propoxycarbonylpentyl)-2-methyl-imidazol-4-yl-, 1-(6-n-Propoxycarbonylhexyl)-2-methyl-imidazol-4-yl-, 1-(7-n-Propoxycarbonylheptyl)-2-methyl-imidazol-4-yl-, 1-Isopropoxycarbonylmethyl2-methyl-imidazol-4-yl-, 1-(2-Isopropoxycarbonylethyl)-2-methyl-imidazol-4-yl-, 1-(3-Isopropoxycarbonylpropyl)-2-methyl-imidazol-4-yl-, 1-(4-Isopropoxycarbonylbutyl)-2-methyl-imidazol-4-yl-, 1-(5-Isopropoxycarbonylpentyl)-2-methyl-imidazol-4-yl-, 1-(6-Isopropoxycarbonylhexyl)-2-methyl-imidazol-4-yl-, 1-(7-Isopropoxycarbonylheptyl)-2-methyl-imidazol-4-yl-, 1-Aminocarbonylmethyl-2-methyl-imidazol-4-yl-, 1-(2-Aminocarbonylethyl)-2-methyl-imidazol-4-yl-, 1-(3-Amino-carbonylpropyl)-2-methyl-imidazol-4-yl-, 1-(4-Aminocarbonylbutyl)-2-methyl-imidazol-4-yl-, 1-(5-Aminocarbonylpentyl)-2-methyl-imidazol-4-yl-, 1-(6-Aminocarbonylhexyl)-2-methyl-imidazol-4-yl-, 1-(7-Aminocarbonylheptyl)-2-methyl-imidazol-4-yl-, 1-Methylaminocarbonylmethyl-2-methyl-imidazol-4-yl-, 1-(2-Methylaminocarbonylethyl)-2-methyl-imidazol-4-yl-, 1-(3-Methylaminocarbonylpropyl)-2-methyl-imidazol-4-yl-, 1-(4-Methylaminocarbonylbutyl)-2-methyl-imidazol-4-yl-, 1-(5-Methylaminocarbonylpentyl)-2-methyl-imidazol-4-yl-, 1-(6-Methylaminocarbonylhexyl)-2-methyl-imidazol-4-yl-, 1-(7-Methylaminocarbonylheptyl)-2-methyl-imidazol-4-yl-, 1-Ethylaminocarbonylmethyl-2-methyl-imidazol-4-yl-, 1-(2-Ethylaminocarbonylethyl)-2-methyl-imidazol-4-yl-, 1-(3-Ethylaminocarbonylpropyl)-2-methyl-imidazol-4-yl-, 1-(4-Ethylaminocarbonylbutyl)-2-methyl-imidazol-4-yl-, 1-(5-Ethylaminocarbonylpentyl)-2-methyl-imidazol-4-yl-, 1-(6-Ethylaminocarbonylhexyl)-2-methyl-imidazol-4-yl-, 1-(7-Ethylaminocarbonylheptyl)-2-methyl-imidazol-4-yl-, 1-n-Propylaminocarbonylmethyl-2-methyl-imidazol-4-yl-, 1-(2-n-Propylaminocarbonylethyl)-2-methyl-imidazol-4-yl-, 1-(3-n-Propylaminocarbonylpropyl)-2-methyl-imidazol-4-yl-, 1-(4-n-Propylaminocarbonylbutyl)-2-methyl-imidazol-4-yl-, 1-(5-n-Propylaminocarbonylpentyl)-2-methyl-imidazol-4-yl-, 1-(6-n-Propylaminocarbonylhexyl)-2-methyl-imidazol-4-yl-, 1-(7-n-Propylaminocarbonylheptyl)-2-methyl-imidazol-4-yl-, 1-Isopropylaminocarbonylmethyl-2-methyl-imidazol-4-yl-, 1-(2-Isopropylaminocarbonylethyl)-2-methyl-imidazol-4-yl-, 1-(3-Isopropylaminocarbonylpropyl)-2-methyl-imidazol-4-yl-, 1-(4-Isopropylaminocarbonylbutyl)-2-methyl-imidazol-4-yl-, 1-(5-Isopropylaminocarbonylpentyl)-2-methyl-imidazol-4-yl-, 1-(6-Isopropylaminocarbonylhexyl)-2-methyl-imidazol-4-yl-, 1-(7-Isopropylaminocarbonylheptyl)-2-methyl-imidazol-4-yl-, 1-Dimethylaminocarbonylmethyl-2-methyl-imidazol-4-yl-, 1-(2-Dimethylaminocarbonylethyl)-2-methyl-imidazol-4-yl-, 1-(3-Dimethylaminocarbonylpropyl)-2-methyl-imidazol-4-yl-, 1-(4-Dimethylaminocarbonylbutyl)-2-methyl-imidazol-4-yl-, 1-(5-Dimethylaminocarbonylpentyl)-2-methyl-imidazol-4-yl-, 1-(6-Dimethylaminocarbonylhexyl)-2-methyl-imidazol-4-yl-, 1-(7-Dimethylaminocarbonylheptyl)-2-methyl-imidazol-4-yl-, 1-Diethylaminocarbonylmethyl-2-methyl-imidazol-4-yl-, 1-(2-Diethylaminocarbonylethyl)-2-methyl-imidazol-4-yl-, 1-(3-Diethylaminocarbonylpropyl)-2-methyl-imidazol-4-yl-, 1-(4-Diethylaminocarbonylbutyl)-2-methyl-imidazol-4-yl-, 1-(5-Diethylaminocarbonylpentyl)-2-methyl-imidazol-4-yl-, 1-(6-Diethylaminocarbonylhexyl)-2-methyl-imidazol-4-yl-, 1-(7-Diethylaminocarbonylheptyl)-2-methyl-imidazol-4-yl-, 1-Di-n-propylaminocarbonylmethyl-2-methyl-imidazol-4-yl-, 1-(2-Di-n-propylaminocarbonylethyl)-2-methyl-imidazol-4-yl-, 1-(3-Di-n-propylaminocarbonylpropyl)-2-methyl-imidazol-4-yl-, 1-(4-Di-n-propylaminocarbonylbutyl)-2-methyl-imidazol-4-yl-, 1-(5-Di-n-propylaminocarbonylpentyl)-2-methyl-imidazol-4-yl-, 1-(6-Di-n-propylaminocarbonylhexyl)-2-methyl-imidazol-4-yl-, 1-(7-Di-n-propylaminocarbonylheptyl)-2-methyl-imidazol-4-yl-, 1-Diisopropylaminocarbonylmethyl-2-methyl-imidazol-4-yl-, 1-(2-Diisopropylaminocarbonylethyl)-2-methyl-imidazol-4-yl-, 1-(3-Diisopropylaminocarbonylpropyl)-2-methyl-imidazol-4-yl-, 1-(4-Diisopropylaminocarbonylbutyl)-2-methyl-imidazol-4-yl-, 1-(5-Diisopropylaminocarbonylpentyl)-2-methyl-imidazol-4-yl-, 1-(6-Diisopropylaminocarbonylhexyl)-2-methyl-imidazol-4-yl-, 1-(7-Diisopropylaminocarbonylheptyl)-2-methyl-imidazol-4-yl-, 1-Morpholinocarbonylmethyl-2-methyl-imidazol-4-yl-, 1-(2-Morpholinocarbonylethyl)-2-methyl-imidazol-4-yl-, 1-(3-Morpholinocarbonylpropyl)-2-methyl-imidazol-4-yl-, 1-(4-Morpholinocarbonylbutyl)-2-methyl-imidazol-4-yl-, 1-(5-Morpholinocarbonylpentyl)-2-methyl-imidazol-4-yl-, 1-(6-Morpholinocarbonylhexyl)-2-methyl-imidazol-4-yl-, 1-(7-Morpholinocarbonylheptyl)-2-methyl-imidazol-4-yl-, 1-Thiomorpholinocarbonylmethyl-2-methyl-imidazol-4-yl-, 1-(2-Thiomorpholinocarbonylethyl)-2-methyl-imidazol-4-yl-, 1-(3-Thiomorpholinocarbonylpropyl)-2-methyl-imidazol-4-yl-, 1-(4-Thiomorpholinocarbonylbutyl)-2-methyl-imidazol-4-yl-, 1-(5-Thiomorpholinocarbonylpentyl)-2-methyl-imidazol-4-yl-, 1-(6-Thiomorpholinocarbonylhexyl)-2-methyl-imidazol-4-yl-, 1-(7-Thiomorpholinocarbonylheptyl)-2-methyl-imidazol-4-yl-, 1-Oxidothiomorpholinocarbonylmethyl-2-methyl-imidazol-4-yl-, 1-(2-Oxidothiomorpholinocarbonylethyl)-2-methyl-imidazol-4-yl-, 1-(3-Oxidothiomorpholinocarbonylpropyl)-2-methyl-imidazol-4-yl-, 1-(4-Oxidothiomorpholinocarbonylbutyl)-2-methyl-imidazol-4-yl-, 1-(5-Oxidothiomorpholinocarbonylpentyl)-2-methyl-imidazol-4-yl-, 1-(6-Oxidothiomorpholinocarbonylhexyl)-2-methyl-imidazol-4-yl-, 1-(7-Oxidothiomorpholinocarbonylheptyl)-2-methyl-imidazol-4-yl-, 1-(2-Hydroxyethyl)-imidazol-4-yl-, 1-(3-Hydroxypropyl)-imidazol-4-yl-, 1-(4-Hydroxybutyl)-imidazol-4-yl-, 1-(2-Methoxyethyl)-imidazol-4-yl-, 1-(3-Methoxypropyl)-imidazol-4-yl-, 1-(4-Methoxybutyl)-imidazol-4-yl-, 1-(2-Ethoxyethyl)-imidazol-4-yl-, 1-(3-Ethoxypropyl)-imidazol-4-yl-, 1-(4-Ethoxybutyl)-imidazol-4-yl-, 1-(2-n-Propoxyethyl)-imidazol-4-yl-, 1-(3-n-Propoxypropyl)-imidazol-4-yl-, 1-(4-n-Propoxybutyl)-imidazol-4-yl-, 1-(2-Isopropoxyethyl)-imidazol-4-yl-, 1-(3-Isopropoxypropyl)-imidazol-4-yl-, 1-(4-Isopropoxybutyl)-imidazol-4-yl-, 1-(2-Imidazol-1-yl-ethyl)-imidazol-4-yl-, 1-(3-Imidazol-1-yl-propyl)-imidazol-4-yl-, 1-(4-Imidazol-1-yl-butyl)-imidazol-4-yl-, 1-(2,2-Diphenyl-ethyl)-imidazol-4-yl-, 1-(3,3-Diphenyl-propyl)-imidazol-4-yl-, 1-(4,4-Diphenyl-butyl)-imidazol-4-yl-, 1-(2-Hydroxyethyl)-2-methyl-imidazol-4-yl-, 1-(3-Hydroxypropyl)-2-methyl-imidazol-4-yl-, 1-(4-Hydroxybutyl)-2-methyl-imidazol-4-yl-, 1-(2-Methoxyethyl)-2-methyl-imidazol-4-yl-, 1-(3-Methoxypropyl)-2-methyl-imidazol-4-yl-, 1-(4-Methoxybutyl)-2-methyl-imidazol-4-yl-, 1-(2-Ethoxyethyl)-2-methyl-imidazol-4-yl-, 1-(3-Ethoxypropyl)-2-methyl-imidazol-4-yl-, 1-(4-Ethoxybutyl)-2-methyl-imidazol-4-yl-, 1-(2-n-Propoxyethyl)-2-methyl-imidazol-4-yl-, 1-(3-n-Propoxypropyl)-2-methyl-imidazol-4-yl-, 1-(4-n-Propoxybutyl)-2-methyl-imidazol-4-yl-, 1-(2-Isopropoxyethyl)-2-methyl-imidazol-4-yl-, 1-(3-Isopropoxypropyl)-2-methyl-imidazol-4-yl-, 1-(4-Isopropoxybutyl)-2-methyl-imidazol-4-yl-, 1-(2-Imidazol-1-yl-ethyl)-2-methyl-imidazol-4-yl-, 1-(3-Imidazol-1-yl-propyl)-2-methyl-imidazol-4-yl-, 1-(4-Imidazol-1-yl-butyl)-2-methyl-imidazol-4-yl-, 1-(2,2-Diphenyl-ethyl)-2-methyl-imidazol-4-yl-, 1-(3,3-Diphenyl-propyl)-2-methyl-imidazol-4-yl-, 1-(4,4-Diphenyl-butyl)-2-methyl-imidazol-4-yl-, 1-[2-(2-Methoxyethoxy)-ethyl]-imidazol-4-yl-, 1-[3-(2-Methoxyethoxy)-propyl]-imidazol-4-yl-, 1-[4-(2-Methoxyethoxy)-butyl]-imidazol-4-yl-, 1-[2-(2-Ethoxyethoxy)-ethyl]-imidazol-4-yl-, 1-[3-(2-Ethoxyethoxy)-propyl]-imidazol-4-yl-, 1-[4-(2-Ethoxyethoxy)-butyl]-imidazol-4-yl-, 1-[2-(2-n-Propoxyethoxy)-ethyl]-imidazol-4-yl-, 1-[3-(2-n-Propoxyethoxy)-propyl]-imidazol-4-yl-, 1-[4-(2-n-Propoxyethoxy)-butyl]-imidazol-4-yl-, 1-[2-(2-Isopropoxyethoxy)-ethyl]-imidazol-4-yl-, 1-[3-(2-Isopropoxyethoxy)-propyl]-imidazol-4-yl-, 1-[4-(2-Isopropoxyethoxy)-butyl]-imidazol-4-yl-, 1-(2-Dimethylaminoethyl)-imidazol-4-yl-, 1-(2-Diethylamino-ethyl)-imidazol-4-yl-, 1-(2-Di-n-propylamino-ethyl)-imidazol-4-yl-, 1-(2-Diisopropylamino-ethyl)-imidazol-4-yl-, 1-(3-Dimethylamino-propyl)-imidazol-4-yl-, 1-(3-Diethylamino-propyl)-imidazol-4-yl-, 1-(3-Di-n-propylamino-propyl)-imidazol-4-yl-, 1-(3-Diisopropylamino-propyl)-imidazol-4-yl-, 1-(4-Dimethylamino-butyl)-imidazol-4-yl-, 1-(4-Diethylamino-butyl)-imidazol-4-yl-, 1-(4-Di-n-propylamino-butyl)-imidazol-4-yl-, 1-(4-Diisopropylamino-butyl)-imidazol-4-yl-, 1-(2-Morpholino-ethyl)-imidazol-4-yl-, 1-(3-Morpholino-propyl)-imidazol-4-yl-, 1-(4-Morpholino-butyl)-imidazol-4-yl-, 1-(2-Pyrrolidino-ethyl)-imidazol-4-yl-, 1-(3-Pyrrolidino-propyl)-imidazol-4-yl-, 1-(4-Pyrrolidino-butyl)-imidazol-4-yl-, 1-(2-Piperidino-ethyl)-imidazol-4-yl-, 1-(3-Piperidino-propyl)-imidazol-4-yl-, 1-(4-Piperidino-butyl)-imidazol-4-yl-, 1-(2-Hexamethylenimino-ethyl)-imidazol-4-yl-, 1-(3-Hexamethylenimino-propyl)-imidazol-4-yl-, 1-(4-Hexamethylenimino-butyl)-imidazol-4-yl-, 1-(2-Thiomorpholino-ethyl)-imidazol-4-yl-, 1-(3-Thiomorpholino-propyl)-imidazol-4-yl-, 1-(4-Thiomorpholino-butyl)-imidazol-4-yl-, 1-[2-(1-Oxido-thiomorpholino)-ethyl]-imidazol-4-yl-, 1-[3-(1-Oxido-thiomorpholino)-propyl]-imid- azol-4-yl- oder 1-[4-(1-Oxido-thiomorpholino)-butyl]-imidazol-4-yl-gruppe,

für $R_3$ die der Ethyl-, n-Propyl-, Isopropyl-, n-Butyl-, Isobutyl-, tert.Butyl-, Cyclopropyl-, Cyclobutyl-, Ethoxy-, n-Propoxy-, Isopropoxy-, Ethylthio-, n-Propylthio- oder Isopropylthiogruppe und

für $R_4$ die der Hydroxycarbonyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 2-Phenylpropionyloxymethoxycarbonyl-, 3-Phenylpropionyloxymethoxycarbonyl-, 4-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-, n-Butyloxycarbonyloxymethoxycarbonyl-, Isobutyloxycarbonyloxymethoxycarbonyl-, tert.Butyloxycarbonyloxymethoxycarbonyl-, n-Pentyloxycarbonyloxymethoxycarbonyl-, Isoamyloxycarbonyloxymethoxycarbonyl-, n-Hexyloxycarbonyloxymethoxycarbonyl-, Cyclopentyloxycarbonyloxymethoxycarbonyl-, Cyclohexyloxycarbonyloxymethoxycarbonyl-, Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, Cyclopentylcarbonyloxymethoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Cyano-, 1H-Tetrazolyl-, 1-Triphenylmethyl-tetrazolyl- oder 2-Triphenylmethyl-tetrazolyl-gruppe in Betracht.

Bevorzugte Verbindungen der obigen allgemeinen Formel I sind diejenigen, in denen

$R_1$ eine Alkylgruppe mit 1 bis 3 Kohlenstoffatomen, ein Wasserstoff-, Fluor-, Chlor- oder Bromatom,

$R_2$ in 6-Stellung eine in 2-Stellung durch eine Phenylgruppe substituierte Oxazol-4-yl- oder Thiazol-4-yl-Gruppe oder

eine gegebenenfalls in 2-Stellung durch eine Alkylgruppe mit 1 bis 6 Kohlenstoffatomen oder durch eine Phenylgruppe substituierte Imidazol-4-yl-Gruppe, wobei die Imidazol-4-yl-Gruppe in 1-Stellung durch eine Alkylgruppe mit 1 bis 7 Kohlenstoffatomen, die in 1-, 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Alkoxy-, Alkoxyalkoxy-, Dialkylamino-, Pyrrolidino-, Piperidino-, Hexamethylenimino-, Morpholino-, Thiomorpholino-, 1-Oxido-thiomorpholino- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine durch Fluor- oder Chloratome, durch Trifluormethyl-, Methyl- oder Methoxygruppen mono- oder disubstituierte Phenylgruppe substituiert ist, oder durch eine durch zwei Phenylgruppen substituierte Alkylgruppe substituiert ist, wobei, soweit nichts anderes erwähnt wurde, die vorstehend erwähnten Alkyl- und Alkoxyteile jeweils 1 bis 3 Kohlenstoffatome enthalten können,
$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und
$R_4$ eine in vivo in eine Carboxygruppe überführbare Gruppe, eine Carboxy-, Cyano-, 1H-Tetrazolyl-, 1-Triphenylmethyltetrazolyl- oder 2-Triphenylmethyl-tetrazolylgruppe bedeuten,
insbesondere diejenigen Verbindungen, in denen
$R_1$ ein Wasserstoff- oder Chloratom oder eine Methylgruppe,
$R_2$ in 6-Stellung eine in 2-Stellung durch eine Phenylgruppe substituierte Oxazol-4-yl- oder Thiazol-4-yl-Gruppe oder eine gegebenenfalls in 2-Stellung durch eine Methylgruppe substituierte Imidazol-4-yl-Gruppe, die in 1-Stellung durch eine Alkylgruppe mit 1 bis 7 Kohlenstoffatomen, die in 1-, 2-, 3-, 4-, 5-, 6- oder 7-Stellung durch eine Carboxy-, Methoxycarbonyl-, Aminocarbonyl-, Methylaminocarbonyl-, Ethylaminocarbonyl-, n-Propylaminocarbonyl-, Dimethylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Methoxy-, 2-Methoxyethoxy-, Dimethylamino-, Diethylamino-, Pyrrolidino-, Piperidino-, Morpholino- oder Imidazol-1-yl-Gruppe substituiert ist, durch eine 2,2,2-Trifluorethyl-, 3,3,3-Trifluorpropyl-, Cyclopropylmethyl-, Cyclobutylmethyl-, Cyclopentylmethyl-, Cyclohexylmethyl-, 4-Fluorbenzyl-, 3-Chlorbenzyl-, 4-Methylbenzyl-, 4-Trifluormethylbenzyl-, 3,5-Dimethoxybenzyl- oder 2,2-Diphenylethylgruppe substituiert ist,
$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und
$R_4$ eine in vivo in eine Carboxygruppe überführbare Gruppe, eine Carboxy- oder 1H-Tetrazolylgruppe bedeuten,
und deren Salze, insbesondere für die pharmazeutische Anwendung deren physiologisch verträglichen Salze mit anorganischen oder organischen Säuren oder Basen.

Besonders bevorzugte Verbindungen der obigen allgemeinen Formel I sind diejenigen, in denen
$R_1$ ein Wasserstoffatom oder eine Methylgruppe,
$R_2$ in 6-Stellung eine in 2-Stellung durch eine Phenylgruppe substituierte Oxazol-4-yl- oder Thiazol-4-yl-Gruppe oder eine gegebenenfalls in 2-Stellung durch eine Methylgruppe substituierte Imidazol-4-yl-Gruppe, die in 1-Stellung durch eine Alkylgruppe mit 1 bis 7 Kohlenstoffatomen, die in 1-, 2-, 3-, 4-, 5-, 6- oder 7-Stellung durch eine Carboxy-, Methoxycarbonyl-, Dimethylaminocarbonyl- oder Morpholinocarbonylgruppe substituiert ist, durch eine Alkylgruppe mit 2 bis 4 Kohlenstoffatomen, die in 2-, 3- oder 4-Stellung durch eine Hydroxy-, Methoxy-, 2-Methoxyethoxy-, Dimethylamino-, Diethylamino-, Pyrrolidino-, Piperidino-, Morpholino- oder Imidazol-1-yl-Gruppe substituiert ist, durch eine 2,2,2-Trifluorethyl-, 3,3,3-Trifluorpropyl-, Cyclopropylmethyl-, Cyclobutylmethyl-, Cyclopentylmethyl-, Cyclohexylmethyl-, oder 4-Fluorbenzylgruppe substituiert ist,
$R_3$ eine Alkylgruppe mit 2 bis 4 Kohlenstoffatomen, eine Ethoxy-, Ethylthio-, Cyclopropyl- oder Cyclobutylgruppe und
$R_4$ eine Carboxy- oder 1H-Tetrazolylgruppe bedeuten,
und deren Salze, insbesondere für die pharmazeutische Anwendung deren physiologisch verträglichen Salze mit anorganischen oder organischen Säuren oder Basen.

Als besonders bevorzugte Verbindungen der obigen allgemeinen Formel I seien folgende erwähnt:

(i) 4'-[(2-n-Propyl-4-methyl-6-(1-(2-N-morpholinoethyl)-imidazol-4-yl)-benzimidazol-1-yl)-methyl]-biphenyl-2-carbonsäure,

(ii) 4'-[(2-n-Propyl-4-methyl-6-(1-(2-methoxyethoxy-2-ethyl)-imidazol-4-yl)-benzimidazol-1-yl)-methyl]-biphenyl-2-carbonsäure,

(iii) 4'-[(2-Ethyl-4-methyl-6-(1-(2-diethylaminoethyl)-imidazol-4-yl)-benzimidazol-1-yl)-methyl]-2-(1H-tetrazol-5-yl)-biphenyl,

(iv) 4'-[(2-Ethyl-4-methyl-6-(1-(3-N-piperidinopropyl)-imidazol-4-yl)-benzimidazol-1-yl)-methyl]-2-(1H-tetrazol-5-yl)-biphenyl,

(v) 4'-[(2-n-Propyl-4-methyl-6-(1-(3-dimethylaminopropyl)-imidazol-4-yl)-benzimidazol-1-yl)-methyl]-biphenyl-2-carbonsäure,

(vi) 4'-[(2-Ethyl-4-methyl-6-(1-(2-N-morpholinoethyl)-imidazol-4-yl)-benzimidazol-1-yl)-methyl]-biphenyl-2-carbonsäure,

(vii) 4'-[(2-Ethyl-4-methyl-6-(1-(2-N-morpholinoethyl)-imidazol-4-yl)-benzimidazol-1-yl)-methyl]-2-(1H-tetrazol-5-yl)-biphenyl,

(viii) 4'-[(2-Ethyl-4-methyl-6-(1-(2-N-pyrrolidinoethyl)-imidazol-4-yl)-benzimidazol-1-yl)-methyl]-biphenyl-2-carbonsäure,

(ix) 4'-[(2-Ethyl-4-methyl-6-(1-(2-N-pyrrolidinoethyl)-imidazol-4-yl)-benzimidazol-1-yl)-methyl]-2-(1H-tetrazol-5-yl)-biphenyl,

(x) 4'-[(2-Ethyl-4-methyl-6-(1-(2-diethylaminoethyl)-imidazol-4-yl)-benzimidazol-1-yl)-methyl]-biphenyl-2-carbonsäure und

(xi) 4'-[(2-Ethyl-4-methyl-6-(1-(3-N-piperidinopropyl)-imidazol-4-yl)-benzimidazol-1-yl)-methyl]-biphenyl-2-carbonsäure

sowie deren Salze, insbesondere für die pharmazeutische Anwendung deren physiologisch verträglichen Salze mit anorganischen oder organischen Säuren oder Basen.

Erfindungsgemäß erhält man die Verbindungen nach folgenden Verfahren:

a) Cyclisierung einer gegebenenfalls im Reaktionsgemisch gebildeten Verbindung der allgemeinen Formel

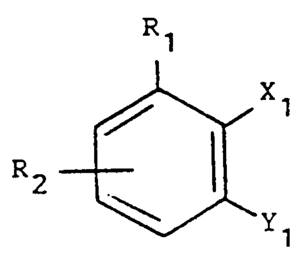

,(II)

in der

$R_1$ und $R_2$ wie eingangs definiert sind,

einer der Reste $X_1$ oder $Y_1$ eine Gruppe der allgemeinen Formel

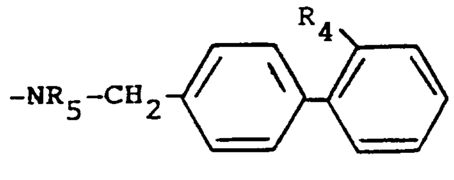

und der andere der Reste $X_1$ oder $Y_1$ eine Gruppe der allgemeinen Formel

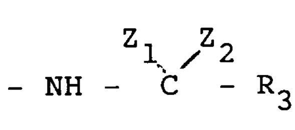

darstellen, wobei
$R_3$ und $R_4$ wie eingangs definiert sind,
$R_5$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch Alkylgruppen mit 1 bis 6 Kohlenstoffatomen substituierte Hydroxy- oder Mercaptogruppen oder
$Z_1$ und $Z_2$, zusammen ein Sauerstoff- oder Schwefelatom,
eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-aminoverbindung gegebenenfalls in Gegenwart einer Carbonsäure der allgemeinen Formel $R_3COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

b) Umsetzung eines Benzimidazols der allgemeinen Formel

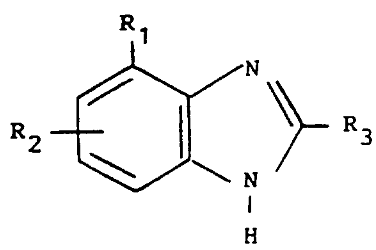
,(III)

in der
$R_1$ bis $R_3$ wie eingangs definiert sind, mit einer Biphenylverbindung der allgemeinen Formel

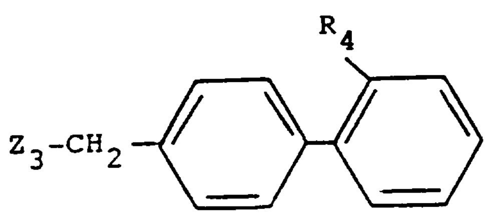
,(IV)

in der
$R_4$ wie eingangs definiert ist und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfosid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Natriumhydrid, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Bei der Umsetzung erhält man vorzugsweise ein Gemisch der 1- und 3-Isomeren, aus welchem anschließend durch Kristallisation oder chromatographisch unter Verwendung eines Trägers wie Kieselgel oder Aluminiumoxid, das entsprechende 1-Isomere abgetrennt wird.

c) Zur Herstellung einer Verbindung der allgemeinen Formel I, in der $R_4$ eine Carboxygruppe darstellt:

Überführung einer Verbindung der allgemeinen Formel

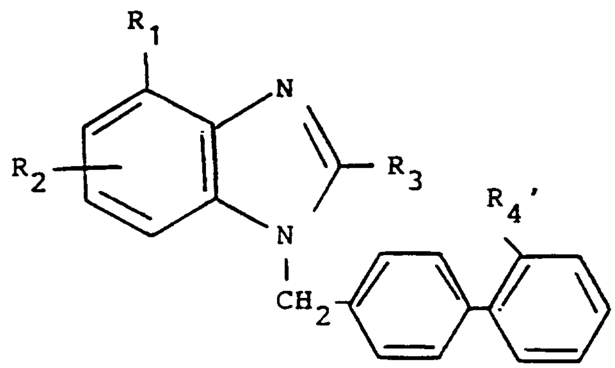

, (V)

in der

$R_1$ bis $R_3$ wie eingangs definiert sind und

$R_4$' eine mittels Hydrolyse, Thermolyse oder Hydrogenolyse in eine Carboxygruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol, Wasser/Dioxan, Methylenchlorid oder Chloroform bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4$' in einer Verbindung der allgemeinen Formel V eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxygruppe übergeführt werden.

Bedeutet $R_4$' in einer Verbindung der allgemeinen Formel V beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_4$' in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und

einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

d) Zur Herstellung einer Verbindung der allgemeinen Formel I, in der $R_4$ eine 1H-Tetrazolylgruppe darstellt:
Abspaltung eines Schutzrestes von einer Verbindung der allgemeinen Formel

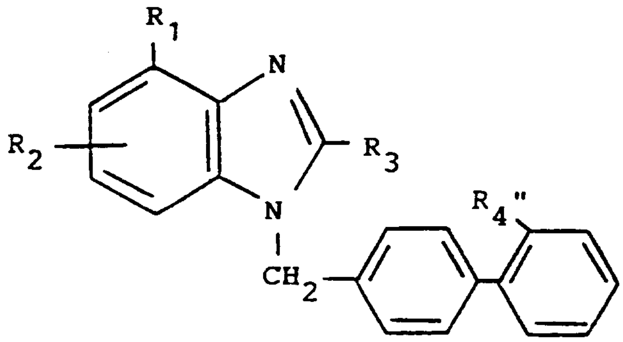

,(VI)

in der
$R_1$, $R_2$ und $R_3$ wie eingangs definiert sind und
$R_4$" eine in 1- oder 3-Stellung durch einen Schutzrest geschützte 1H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_4$ eine 1H-Tetrazolylgruppe darstellt:
Umsetzung einer Verbindung der allgemeinen Formel

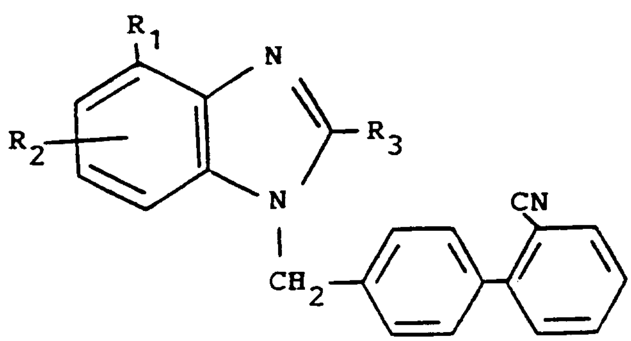

,(VII)

in der
$R_1$ bis $R_3$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie

Natriumazid hergestellt werden, erhaltene Tetrazolidsalz anschließend durch Ansäuern mit einer verdünnten Säure wie 2N Salzsäure oder 2N Schwefelsäure freigesetzt.

Erhält man erfindungsgemäß eine Verbindung der allgemeinen Formel I, in der $R_4$ eine Carboxygruppe darstellt, so kann diese mittels Veresterung in eine entsprechende Verbindung der allgemeinen Formel I, in der $R_4$ eine in vivo in eine Carboxygruppe überführbare Gruppe darstellt, übergeführt werden oder
erhält man erfindungsgemäß eine Verbindung der allgemeinen Formel I, in der $R_2$ eine Imidazol-4-yl-gruppe darstellt, die in 1-Stellung durch eine Alkylgruppe mit 2 bis 4 Kohlenstoffatomen substituiert ist, wobei die Alkylgruppe in 2-, 3- oder 4-Stellung durch eine Alkoxy- oder Alkoxyalkoxygruppe substituiert ist, so kann diese mittels Etherspaltung in eine entsprechende Verbindung der allgemeinen Formel I übergeführt werden, in der $R_2$ eine Imidazol-4-yl-gruppe darstellt, die in 1-Stellung durch eine Alkylgruppe mit 2 bis 4 Kohlenstoffatomen substituiert ist, wobei die Alkylgruppe in 2-, 3- oder 4-Stellung durch eine Hydroxygruppe substituiert ist.

Die Überführung einer Carboxylgruppe in eine Gruppe, die in vivo metabolisch in eine Carboxygruppe umgewandelt wird, erfolgt zweckmäßigerweise durch Veresterung mit einem entsprechenden Alkohol oder mit einem entsprechenden reaktionsfähigen Acylderivat zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel gegebenenfalls in Gegenwart eines die Säure aktivierenden oder wasserentziehenden Mittels wie Thionylchlorid, mit deren Anhydriden, Estern oder Halogeniden gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Die nachträgliche Etherspaltung erfolgt hydrolytisch in einem wässrigen Lösungsmittel, z. B. in Wasser, Isopropanol/Wasser, Tetrahydrofuran/Wasser oder Dioxan/Wasser, in Gegenwart einer Säure wie Jodwasserstoffsäure oder Bromwasserstoffsäure, vorzugsweise jedoch unter Einwirkung einer Lewis-Säure wie Bortrifluorid, Bortribromid, Bortrichlorid, Dimethylborbromid oder Aluminiumtrichlorid in einem geeigneten Lösungsmittel wie Dichlormethan oder Chloroform, oder mit Hilfe von Bromtrimethylsilan, Jodtrimethylsilan oder Chlortrimethylsilan/Natriumjodid in einem geeigneten Lösungsmittel wie Acetonitril, Dichlormethan oder Chloroform bei Temperaturen zwischen 0 und 100°C, vorzugsweise bei 20°C, und anschließender wässriger Aufarbeitung.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Von einem so erhaltenen 1-, 3-Isomerengemisch einer Verbindung der allgemeinen Formel I wird anschließend vorzugsweise chromatographisch unter Verwendung eines Trägers wie Kieselgel oder Aluminiumoxid das 1-Isomere abgetrennt.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis VII sind teilweise literaturbekannt oder man erhält diese nach literaturbekannten Verfahren.

So erhält man beispielsweise eine Verbindung der allgemeinen Formel II durch Alkylierung einer entsprechenden o-Aminoacylamino-Verbindung mit einer Verbindung der allgemeinen Formel IV. Die hierfür erforderliche o-Amino-acylamino-Verbindung erhält man durch Reduktion einer entsprechenden o-Nitro-acylamino-Verbindung, welche man ihrerseits durch Nitrierung eines entsprechenden Acylamino-acetophenons, anschließende Überführung des erhaltenen entsprechenden o-Nitro-acylamino-acetophenons in das entsprechende ω-Bromacetophenon, anschließende Cyclisierung des ω-Brom-acetophenons mit einem entsprechenden Säureamid und anschließende Reduktion der Nitrogruppe erhält. Vor der Reduktion der Nitrogruppe kann eine so erhaltene Oxazol-4-yl-Verbindung mittels einem entsprechenden Amin, vorzugsweise mit Ammoniak, unter Druck in die entsprechende Imidazol-4-yl-Verbindung übergeführt werden oder eine so erhaltene in 1-Stellung unsubstituierte Imidazol-4-yl-Verbindung mittels Alkylierung in eine entsprechend in 1-Stellung alkylierte Imidazol-4-yl-Verbindung übergeführt werden.

Eine Ausgangsverbindung der allgemeinen Formel III erhält man durch Reduktion und Cyclisierung einer vorstehend beschrieben o-Nitro-acylamino-Verbindung.

Eine Ausgangsverbindung der allgemeinen Formeln V, VI und VII erhält man durch Umsetzung einer Verbindung der allgemeinen Formel III mit einer entsprechenden Verbindung der allgemeinen Formel IV.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[(2-n-Propyl-4-methyl-6-(1-methyl-imidazol-4-yl)-benzimidazol-1-yl)-methyl]-biphenyl-2-carbonsäure (nicht erfindungsgemäß),

B = 4'-[(2-n-Propyl-4-methyl-6-(1-isopropyl-imidazol-4-yl)-benzimidazol-1-yl)-methyl]-2-(1H-tetrazol-5-yl)-biphenyl (nicht erfindungsgemäß),

C = 4'-[(2-n-Propyl-4-methyl-6-(2-methyl-oxazol-4-yl)-benzimidazol-1-yl)-methyl]-biphenyl-2-carbonsäure,

D = 4'-[(2-Ethoxy-6-(1-isopropyl-imidazol-4-yl)-benzimidazol-1-yl)-methyl]-biphenyl-2-carbonsäure,

E = 4'-[(2-n-Propyl-4-methyl-6-(1-cycloheptyl-imidazol-4-yl)-benzimidazol-1-yl)-methyl]-biphenyl-2-carbonsäure,

F = 4'-[(2-n-Propyl-4-methyl-6-(1-aminocarbonylmethyl-imidazol-4-yl)-benzimidazol-1-yl)-methyl]-biphenyl-2-carbonsäure und

G = 4'-[(2-n-Propyl-4-methyl-6-(1-(3-dimethylaminopropyl)-imidazol-4-yl)-benzimidazol-1-yl)-methyl]-biphenyl-2-carbonsäure-dihydrochlorid-pentahydrat

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$125^I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis E zeigen in dem beschriebenen Test folgende $IC_{50}$-Werte:

| Substanz | $IC_{50}$ [nM] |
|---|---|
| A | 1,2 |
| B | 1,5 |
| C | 40 |
| D | 10 |
| E | 15 |
| F | 3,4 |
| G | 1,3 |

Aufgrund ihrer pharmakologischen Eigenschaften, nämlich einer blutdrucksenkenden Wirkung mit einer diuretischen/saluretischen Komponente, eignen sich die neuen Verbindungen und deren physiologisch verträgliche Salze zur Behandlung der Hypertonie und Herzinsuffizienz, ferner zur Behandlung der chronischen Niereninsuffizienz, zur Behandlung ischämischer peripherer (beispielsweise Raynaud's Syndrom) sowie cerebrovaskulärer Durchblutungsstörungen, der myokardialen Ischämie (Angina), zur Prävention der Herzinsuffizienzprogression nach Myokard-Infarkt, zur Behandlung der diabetischen Nephro- und Retinopathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose, der diabetischen Angiopathie, der Hyperuricämie und der Gicht. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel A (nicht erfindungsgemäß)

4'-[(2-n-Propyl-4-methyl-6-(1-methyl-imidazol-4-yl)-benzimidazol-1-yl)-methyl]-biphenyl-2-carbonsäure

a) 3-Methyl-4-butyrylamino-5-nitro-acetophenon

32,6 g (148 mMol) 3-Methyl-4-butyrylamino-acetophenon werden unter Rühren portionsweise bei -15°C in 300 ml rauchende Salpetersäure eingetragen und weitere 30 Minuten bei -15°C gerührt. Das Reaktionsgemisch wird dann

unter Rühren auf 3 l Eis gegossen, das ausgefallene Rohprodukt abgesaugt, mit 400 ml Wasser gewaschen, getrocknet und durch Umkristallisieren aus Ethanol/Diethylether (1:1) gereinigt.
Ausbeute: 23,8 g (61,0 % der Theorie),
$R_f$-Wert: 0,32 (Kieselgel; Methylenchlorid)
$R_f$-Wert: 0,48 (Kieselgel; Methylenchlorid/Methanol = 50:1)

b) 3-Methyl-4-butyrylamino-5-nitro-ω-bromacetophenon

Zu einer Lösung von 23,8 g (90 mMol) 3-Methyl-4-butyrylamino-5-nitro-acetophenon in 900 ml Dichlormethan wird bei Raumtemperatur unter Rühren eine Lösung von 16,0 g (200 mMol) Brom in 140 ml Dioxan so langsam zugetropft, daß stets vollständige Entfärbung des Reaktionsgemisches stattfindet. Anschließend wird weitere zwei Stunden gerührt, dann das Reaktionsgemisch im Vakuum bis zur Trockne eingeengt, der so erhaltene Rückstand mit ca. 20 ml Dichlormethan/Diethylether (1:1) verrieben, abgesaugt und dann getrocknet. Man erhält so 23 g (74 % der Theorie) 3-Methyl-4-butyrylamino-5-nitro-ω-bromacetophenon, in dem noch ca. 10 % Ausgangsmaterial enthalten sind. Das Produkt wird ohne weitere Reinigung weiter umgesetzt.
$R_f$-Wert: 0,69 (Kieselgel; Methylenchlorid/Methanol = 50:1)
$R_f$-Wert: 0,84 (Kieselgel; Methylenchlorid/Methanol = 9:1)

c) 2-Butyrylamino-3-nitro-5-(imidazol-4-yl)-toluol

Eine Lösung von 6,8 g (20 mMol) 3-Methyl-4-butyrylamino-5-nitro-ω-bromacetophenon in 20 ml Formamid wird 2 Stunden lang auf 140°C erhitzt. Die abgekühlte Lösung wird dann in ca. 50 ml 1 N Ammoniak gegossen und ca. 15 Minuten lang gerührt. Das ausgefallene Rohprodukt wird abgesaugt, mit ca. 50 ml Wasser gewaschen und getrocknet. Man erhält so 4,4 g (75 % der Theorie) des Produktes, das ohne weitere Reinigung weiter umgesetzt wird.
$R_f$-Wert: 0,29 (Kieselgel; Methylenchlorid/Methanol = 9:1)

d) 2-Butyrylamino-3-nitro-5-(1-methyl-imidazol-4-yl)-toluol

Zu einer Lösung von 2,5 g (8,7 mMol) 2-Butyrylamino-3-nitro-5-(imidazol-4-yl)-toluol und 5,2 g (30 mMol) Kaliumcarbonat-Dihydrat in 30 ml Dimethylsulfoxid werden 1,3 g (9,5 mMol) Methyljodid bei Raumtemperatur zugetropft und anschließend 2 Stunden lang gerührt. Das Reaktionsgemisch wird dann in ca. 150 ml Wasser eingerührt und anschließend viermal mit je 25 ml Essigester extrahiert. Die organischen Extrakte werden mit ca. 30 ml Wasser gewaschen, getrocknet und eingeengt. Das so erhaltene Rohprodukt wird durch Säulenchromatographie (300 g Kieselgel, Laufmittel: Methylenchlorid/Methanol = 30:1) gereinigt.
Ausbeute: 640 mg (24 % der Theorie),
$R_f$-Wert: 0,54 (Kieselgel; Methylenchlorid/Methanol = 9:1)

e) 2-Butyrylamino-3-amino-5-(1-methyl-imidazol-4-yl)-toluol

640 mg (2,1 mMol) 2-Butyrylamino-3-nitro-5-(1-methyl-imidazol-4-yl)-toluol werden in 30 ml Methanol nach Zugabe von ca. 200 mg Palladium/Kohle (20 %) bei Raumtemperatur und einem Wasserstoff-Druck von 5 bar hydriert. Nach vollständiger Wasserstoffaufnahme wird vom Katalysator abfiltriert und das Filtrat eingeengt. Das so erhaltene Rohprodukt wird ohne weitere Reinigung weiter umgesetzt.
Ausbeute: 600 mg (100 % der Theorie),
$R_f$-Wert: 0,23 (Kieselgel; Methylenchlorid/Methanol = 9:1)

f) 2-n-Propyl-4-methyl-6-(1-methyl-imidazol-4-yl)-benzimidazol

600 mg (2,1 mMol) 2-Butyrylamino-3-amino-5-(1-methyl-imidazol-4-yl)-toluol werden in 10 ml Eisessig eine Stunde lang zum Rückfluß erhitzt. Dann wird im Vakuum zur Trockene eingedampft, der Rückstand mit ca. 15 ml Wasser versetzt, mit Ammoniak alkalisch gestellt und viermal mit je ca. 10 ml Essigester extrahiert. Die organischen Extrakte werden mit ca. 15 ml Wasser gewaschen, getrocknet und schließlich eingeengt. Das so erhaltene Rohprodukt wird ohne weitere Reinigung weiter umgesetzt.
Ausbeute: 420 mg (79 % der Theorie),
$R_f$-Wert: 0,37 (Kieselgel; Methylenchlorid/Methanol = 9:1)

g) 4'-[(2-n-Propyl-4-methyl-6-(1-methyl-imidazol-4-yl)-benzimidazol-1-yl)-methyl]-biphenyl-2-carbonsäure-tert.butylester

Zu einer Lösung von 200 mg (0,79 mMol) 2-n-Propyl-4-methyl-6-(1-methyl-imidazol-4-yl)-benzimidazol und 90 mg (0,8 mMol) Kalium-tert.butylat in 5 ml Dimethylsulfoxid werden 280 mg (0,8 mMol) 4'-Brommethyl-biphenyl-2-carbonsäure-tert.butylester gegeben und das Gemisch 90 Minuten lang bei Raumtemperatur gerührt, dann in ca. 40 ml Wasser eingerührt, viermal mit je ca. 10 ml Essigester extrahiert, dann die organischen Extrakte mit 10 ml Wasser gewaschen, getrocknet und zur Trockne eingeengt. Das so erhaltene Rohprodukt wurde durch Säulenchromatographie (100 g Kieselgel, Laufmittel; Dichlormethan/Methanol = 30:1) gereinigt.
Ausbeute: 230 mg (56 % der Theorie),
$R_f$-Wert: 0,61 (Kieselgel; Methylenchlorid/Methanol = 9:1)

h) 4'-[(2-n-Propyl-4-methyl-6-(1-methyl-imidazol-4-yl)-benzimidazol-1-yl)-methyl]-biphenyl-2-carbonsäure

Eine Lösung von 230 mg (0,44 mMol) 4'-[(2-n-Propyl-4-methyl-6-(1-methyl-imidazol-4-yl)-benzimidazol-1-yl)-methyl]-biphenyl-2-carbonsäure-tert.butylester und 2 ml Trifluoressigsäure in 10 ml Dichlormethan wurde bei Raumtemperatur über Nacht gerührt und anschließend zur Trockne eingeengt. Der Rückstand wurde in ca. 5 ml verdünnter Natronlauge gelöst, die Lösung mit Essigsäure neutralisiert, der danach ausgefallene Niederschlag abgesaugt, mit Wasser gewaschen und getrocknet.
Ausbeute: 120 mg (59 % der Theorie),
Schmelzpunkt: 293-295°C
$R_f$-Wert: 0,39 (Kieselgel; Methylenchlorid/Methanol = 9:1)

Beispiel B (nicht erfindungsgemäß)

4'-[(2-n-Propyl-4-methyl-6-(1-methyl-imidazol-4-yl)-benzimidazol-1-yl)-methyl]-2-(1H-tetrazol-5-yl)-biphenyl-hydrat

a) 4'-[(2-n-Propyl-4-methyl-6-(1-methylimidazol-4-yl)-benzimidazol-1-yl)-methyl]-2-cyano-biphenyl

Zu einer Lösung von 200 mg (0,79 mMol) 2-n-Propyl-4-methyl-6-(1-methyl-imidazol-4-yl)-benzimidazol und 90 mg (0,8 mMol) Kalium-tert.butylat in 6 ml Dimethylsulfoxid werden 218 mg (0,8 mMol) 4'-Brommethyl-2-cyano-biphenyl gegeben und das Gemisch 14 Stunden lang bei Raumtemperatur gerührt. Anschliessend wird in ca. 40 ml Wasser eingerührt, viermal mit je ca. 10 ml Essigester extrahiert, die organischen Extrakte mit ca. 10 ml Wasser gewaschen, getrocknet und zur Trockne eingeengt. Das so erhaltene Rohprodukt wird durch Säulenchromatographie (100 g Kieselgel; Laufmittel: Dichlormethan/Ethanol = 50:1) gereinigt.
Ausbeute: 240 mg (67 % der Theorie),
$R_f$-Wert: 0,38 (Kieselgel; Methylenchlorid/Ethanol = 19:1)

b) 4'-[(2-n-Propyl-4-methyl-6-(1-methyl-imidazol-4-yl)-benzimidazol-1-yl)-methyl]-2-(1H-tetrazol-5-yl)-biphenyl-hydrat

Eine Lösung aus 222 mg (0,5 mMol) 4'-[(2-n-Propyl-4-methyl-6-(1-methyl-imidazol-4-yl)-benzimidazol-1-yl)-methyl]-2-cyano-biphenyl, 660 mg (10 mMol) Natriumazid und 540 mg (10 mMol) Ammoniumchlorid in 12 ml reinem Dimethylformamid wird 18 Stunden auf 140°C erhitzt. Die Lösung wird dann annähernd bis zur Trockne eingeengt und das Produkt durch Säulenchromatographie isoliert (60 g Kieselgel, Laufmittel: Dichlormethan mit 10 % Ethanol). Das so erhaltene Produkt wird in ca. 10 ml verdünnter Ammoniak-Lösung aufgenommen und die Lösung dann mit Essigsäure auf pH 6 eingestellt. Es bildet sich ein schmieriger Niederschlag, der nach Zugabe von wenig Essigester und mehrstündigem Rühren kristallin wird. Das kristalline Produkt wird abgesaugt, mit ca. 5 ml Wasser gewaschen und getrocknet.
Ausbeute: 61,0 mg (24,0 % der Theorie),
Schmelzpunkt: 255-257°C
$C_{29}H_{28}N_8 \times H_2O$ (506,62)

| | | | | | | |
|---|---|---|---|---|---|---|
| Ber.: | C | 68,75 | H | 5,97 | N | 22,12 |
| Gef.: | | 68,90 | | 5,97 | | 22,03 |

$R_f$-Wert: 0,24 (Kieselgel; Methylenchlorid/Methanol = 9:1)

Beispiel 1

4'-[(2-n-Propyl-4-methyl-6-(1-cyclopentylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-cyclopentylmethyl-imidazol-4-yl)-benzimidazol-1-yl)-methyl]-biphenyl-2-carbonsäure-tert.butylester und Trifluoressigsäure in Methylenchlorid.

Beispiel 2

4'-[(2-n-Propyl-4-methyl-6-(1-cyclohexylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-cyclohexylmethyl-imidazol-4-yl)-benzimidazol-1-yl)-methyl]-biphenyl-2-carbonsäure-tert.butylester und Trifluoressigsäure in Methylenchlorid.

Beispiel 3

4'-[(2-n-Propyl-4-methyl-6-(1-(4-fluorbenzyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(4-fluorbenzyl)-imidazol-4-yl)-benzimidazol-1-yl)-methyl]-biphenyl-2-carbonsäure-tert.butylester und Trifluoressigsäure in Methylenchlorid.

Analog Beispiel 5 können folgende Verbindungen erhalten werden:
4'-[(2-n-Propyl-4-methyl-6-(1-(3-chlorbenzyl)-imidazol-4-yl)-benzimidazol-1-yl)-methyl]-biphenyl-2-carbonsäure
4'-[(2-n-Propyl-4-methyl-6-(1-(3,5-dimethoxybenzyl)-imidazol-4-yl)-benzimidazol-1-yl)-methyl]-biphenyl-2-carbonsäure
4'-[(2-n-Propyl-4-methyl-6-(1-(4-methylbenzyl)-imidazol-4-yl)-benzimidazol-1-yl)-methyl]-biphenyl-2-carbonsäure
4'-[(2-n-Propyl-4-methyl-6-(1-(4-trifluormethyl-benzyl)-imidazol-4-yl)-benzimidazol-1-yl)-methyl]-biphenyl-2-carbonsäure

Beispiel 4

4'-[(2-n-Propyl-4-methyl-6-(1-(2-phenylethyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(2-phenylethyl)-imidazol-4-yl)-benzimidazol-1-yl)-methyl]-biphenyl-2-carbonsäure-tert.butylester und Trifluoressigsäure in Methylenchlorid.

Beispiel 5

4'-[(2-n-Propyl-4-methyl-6-(1-(2,2,2-trifluorethyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(2,2,2-trifluorethyl)-imidazol-4-yl)-benzimidazol-1-yl)-methyl]-biphenyl-2-carbonsäure-tert.butylester und Trifluoressigsäure in Methylenchlorid.

Beispiel 6

4'-[(2-n-Propyl-4-methyl-6-(1-(3,3,3-trifluorpropyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(3,3,3-trifluorpropyl)-imidazol-4-yl)-benzimidazol-1-yl)-methyl]-biphenyl-2-carbonsäure-tert.butylester und Trifluoressigsäure in Methylenchlorid.

Beispiel 7

4'-[(2-n-Propyl-4-methyl-6-(1-aminocarbonylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-aminocarbonylmethyl-imidazol-4-yl)-benzimidazol-1-yl)-methyl]-biphenyl-2-carbonsäure-tert.butylester und Trifluoressigsäure in Methylenchlorid.

Beispiel 8

4'-[(2-n-Propyl-4-methyl-6-(1-cyclobutylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-cyclobutylmethyl-imidazol-4-yl)-benzimidazol-1-yl)-methyl]-biphenyl-2-carbonsäure-tert.butylester und Trifluoressigsäure in Methylenchlorid.

Beispiel 9

4'-[(2-n-Propyl-4-methyl-6-(1-cyclopropylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-cyclopropylmethyl-imidazol-4-yl)-benzimidazol-1-yl)-methyl]-biphenyl-2-carbonsäure-tert.butylester und Trifluoressigsäure in Methylenchlorid.
Ausbeute: 59,0 % der Theorie,
Schmelzpunkt: 279-280°C
$C_{32}H_{32}N_4O_2$ (504,64)

| | | | | | | |
|---|---|---|---|---|---|---|
| Ber.: | C | 76,16 | H | 6,39 | N | 11,10 |
| Gef.: | | 76,41 | | 6,37 | | 11,20 |

$R_f$-Wert: 0,44 (Kieselgel; Methylenchlorid/Methanol = 9:1)
Massenspektrum: m/e = 504

Beispiel 10

4'-[(2-n-Propyl-4-methyl-6-(1-cyclopropylmethyl-imidazol-4-yl)-benzimidazol-1-yl)-methyl]-2-(1H-tetrazol-5-yl)-biphenyl

a) 4'-[(2-n-Propyl-4-methyl-6-(1-cyclopropylmethyl-imidazol-4-yl)-benzimidazol-1-yl)-methyl]-2-(2-triphenylmethyltetrazol-5-yl)-biphenyl

Zu einer Lösung von 300 mg (1,0 mMol) 2-n-Propyl-4-methyl-6-(1-cyclopropylmethyl-imidazol-4-yl)-benzimidazol und 110 mg (1,0 mMol) Kalium-tert.butylat in 20 ml Dimethylsulfoxid werden 560 mg (1,0 mMol) 4'-Brommethyl-2-(2-triphenylmethyltetrazol-5-yl)-biphenyl gegeben und das Gemisch 16 Stunden lang bei Raumtemperatur gerührt, dann in ca. 120 ml Wasser eingerührt und viermal mit je ca. 15 ml Essigester extrahiert. Die organischen Extrakte werden mit ca. 30 ml Wasser gewaschen, getrocknet und dann bis zur Trockne eingeengt. Das so erhaltene Rohprodukt wird durch Säulenchromatographie (100 g Kieselgel, Laufmittel: Methylenchlorid/Methanol = 30:1) gereinigt.
Ausbeute: 460 mg (60 % der Theorie),
$R_f$-Wert: 0,78 (Kieselgel; Methylenchlorid/Methanol = 9:1)

b) 4'-[(2-n-Propyl-4-methyl-6-(1-cyclopropylmethyl-imidazol-4-yl)-benzimidazol-1-yl)-methyl]-2-(1H-tetrazol-5-yl)-biphenyl

Ein Gemisch aus 460 mg (0,6 mMol) 4'-[(2-n-Propyl-4-methyl-6-(1-cyclopropylmethyl-imidazol-4-yl)-benzimidazol-1-yl)-methyl]-2-(2-triphenylmethyl-tetrazol-5-yl)-biphenyl und 10 ml gesättigter methanolischer Salzsäure wird eine Stunde lang bei Raumtemperatur gerührt. Anschließend wird bis zur Trockne eingeengt, der Rückstand in verdünnter Ammoniak-Lösung gelöst und mit Ether gewaschen. Die wäßrige Phase wird mit Essigsäure auf pH 5 bis 6 eingestellt und anschließend der ausgefallene Feststoff abgesaugt. Das so erhaltene Rohprodukt wird durch Säulenchromatographie (100 g Kieselgel, Laufmittel: Methylenchlorid/Methanol = 15:1) gereinigt.
Ausbeute: 130 mg (41 % der Theorie),
Schmelzpunkt: amorph
$C_{32}H_{32}N_8$ (528,67)
$R_f$-Wert: 0,32 (Kieselgel; Methylenchlorid/Methanol = 9:1)
Massenspektrum: m/e = 528

Beispiel 11

4'-[(2-n-Propyl-4-methyl-6-(1-cyclobutylmethyl-imidazol-4-yl)-benzimidazol-1-yl)-methyl]-2-(1H-tetrazol-5-yl)-biphenyl

Hergestellt analog Beispiel 10 aus 4'-[(2-n-Propyl-4-methyl-6-(1-cyclobutylmethyl-imidazol-4-yl)-benzimidazol-1-yl)-methyl]-2-(2-triphenylmethyl-tetrazol-5-yl)-biphenyl und Natriumazid in Dimethylformamid.

Beispiel 12

4'-[(2-n-Propyl-4-methyl-6-(2-methyl-oxazol-4-yl)-benzimidazol-1-yl)-methyl]-biphenyl-2-carbonsäure

Hergestellt analog Beispiel A aus 4'-[(2-n-Propyl-4-methyl-6-(2-methyl-oxazol-4-yl)-benzimidazol-1-yl)-methyl]-biphenyl-2-carbonsäure-tert.butylester und Trifluoressigsäure in Methylenchlorid.
Ausbeute: 67,0 % der Theorie,
Schmelzpunkt: 241-243°C
$C_{29}H_{27}N_3O_3$ (465,56)

| | | | | | | |
|---|---|---|---|---|---|---|
| Ber.: | C | 74,82 | H | 5,85 | N | 9,03 |
| Gef.: | | 74,65 | | 5,98 | | 8,85 |

$R_f$-Wert: 0,27 (Kieselgel; Methylenchlorid/Ethanol = 19:1)

Beispiel 13

4'-[(2-n-Propyl-4-methyl-6-(2-methyl-oxazol-4-yl)-benzimidazol-1-yl)-methyl]-2-(1H-tetrazol-5-yl)-biphenyl

Hergestellt analog Beispiel 10 aus 4'-[(2-n-Propyl-4-methyl-6-(2-methyl-oxazol-4-yl)-benzimidazol-1-yl)-methyl]-2-(2-triphenylmethyl-tetrazol-5-yl)-biphenyl und Natriumazid in Dimethylformamid.

Beispiel 14

4'-[(2-n-Propyl-4-methyl-6-(2-phenyl-oxazol-4-yl)-benzimidazol-1-yl)-methyl]-biphenyl-2-carbonsäure

Hergestellt analog Beispiel A aus 4'-[(2-n-Propyl-4-methyl-6-(2-phenyl-oxazol-4-yl)-benzimidazol-1-yl)-methyl]-biphenyl-2-carbonsäure-tert.butylester und Trifluoressigsäure in Methylenchlorid.
Ausbeute: 87,0 % der Theorie,
Schmelzpunkt: 281-283°C
$C_{34}H_{29}N_3O_3$ (527,63)

| | | | | | | |
|---|---|---|---|---|---|---|
| Ber.: | C | 77,40 | H | 5,54 | N | 7,96 |
| Gef.: | | 77,09 | | 5,71 | | 7,76 |

$R_f$-Wert: 0,18 (Kieselgel; Methylenchlorid/Ethanol = 19:1)

Beispiel 15

4'-[(2-n-Propyl-4-methyl-6-(2-phenyl-oxazol-4-yl)-benzimidazol-1-yl)-methyl]-2-(1H-tetrazol-5-yl)-biphenyl

Hergestellt analog Beispiel 10 aus 4'-[(2-n-Propyl-4-methyl-6-(2-phenyl-oxazol-4-yl)-benzimidazol-1-yl)-methyl]-2-(2-triphenylmethyl-tetrazol-5-yl)-biphenyl und Natriumazid in Dimethylformamid.

Beispiel 16

4'-[(2-Ethoxy-6-(1-isopropyl-imidazol-4-yl)-benzimidazol-1-yl)-methyl]-2-(1H-tetrazol-5-yl)-biphenyl

Hergestellt analog Beispiel 10 aus 4'-[(2-Ethoxy-6-(1-isopropyl-imidazol-4-yl)-benzimidazol-1-yl)-methyl]-2-(2-triphenylmethyl-tetrazol-5-yl)-biphenyl.
Ausbeute: 69,0 % der Theorie,
Schmelzpunkt: 175-178°C
$C_{29}H_{28}N_8O$ (504,61)

| | | | | | | |
|---|---|---|---|---|---|---|
| Ber.: | C | 69,03 | H | 5,59 | N | 22,21 |
| Gef.: | | 68,85 | | 5,58 | | 21,97 |

$R_f$-Wert: 0,27 (Kieselgel; Methylenchlorid/Ethanol = 9:1)
Massenspektrum: m/e = 504

Beispiel 17

4'-[(2-Ethoxy-6-(1-isopropyl-imidazol-4-yl)-benzimidazol-1-yl)-methyl]-biphenyl-2-carbonsäure

Hergestellt analog Beispiel A aus 4'-[(2-Ethoxy-6-(1-isopropyl-imidazol-4-yl)-benzimidazol-1-yl)-methyl]-biphenyl-2-carbonsäure-tert.butylester und Trifluoressigsäure in Methylenchlorid.
Ausbeute: 38,0 % der Theorie,
Schmelzpunkt: 220-223°C
$C_{29}H_{28}N_4O_3$ (480,58)

| | | | | | | |
|---|---|---|---|---|---|---|
| Ber.: | C | 72,48 | H | 5,87 | N | 11,66 |
| Gef.: | | 72,36 | | 6,05 | | 11,41 |

$R_f$-Wert: 0,26 (Kieselgel; Methylenchlorid/Methanol = 9:1)
Massenspektrum: m/e = 480

Beispiel 18

4'-[(2-Ethoxy-5-(1-isopropyl-imidazol-4-yl)-benzimidazol-1-yl)-methyl]-2-(1H-tetrazol-5-yl)-biphenyl

Hergestellt analog Beispiel 10 aus 4'-[(2-Ethoxy-5-(1-isopropyl-imidazol-4-yl)-benzimidazol-1-yl)-methyl]-2-(2-triphenylmethyl-tetrazol-5-yl)-biphenyl.
Ausbeute: 44,0 % der Theorie,
Schmelzpunkt: amorph
$C_{29}H_{28}N_8O$ (504,61)
$R_f$-Wert: 0,24 (Kieselgel; Methylenchlorid/Ethanol = 9:1)
Massenspektrum: m/e = 504

Beispiel 19

4'-[(2-n-Propyl-4-methyl-6-(1-cycloheptyl-imidazol-4-yl)-benzimidazol-1-yl)-methyl]-biphenyl-2-carbonsäure

Hergestellt analog Beispiel A aus 4'-[(2-n-Propyl-4-methyl-6-(1-cycloheptyl-imidazol-4-yl)-benzimidazol-1-yl)-methyl]-biphenyl-2-carbonsäure-tert.butylester und Trifluoressigsäure in Methylenchlorid.
Ausbeute: 79,0 % der Theorie,
Schmelzpunkt: ab 190°C (Zers.)
$C_{35}H_{38}N_4O_2$ (546,71)
$R_f$-Wert: 0,36 (Kieselgel; Methylenchlorid/Methanol = 9:1)
Massenspektrum: m/e = 546

Beispiel 20

4'-[(2-n-Propyl-4-methyl-6-(1-cycloheptyl-imidazol-4-yl)-benzimidazol-1-yl)-methyl]-2-(1H-tetrazol-5-yl)-biphenyl

Hergestellt analog Beispiel 10 aus 4'-[(2-n-Propyl-4-methyl-6-(1-cycloheptyl-imidazol-4-yl)-benzimidazol-1-yl)-methyl]- 2-(2-triphenylmethyl-tetrazol-5-yl)-biphenyl.
Ausbeute: 27,0 % der Theorie,
Schmelzpunkt: 198-201°C
$C_{35}H_{38}N_8$ (570,75)
$R_f$-Wert: 0,48 (Kieselgel; Methylenchlorid/Methanol = 9:1)
Massenspektrum: m/e = 570

Beispiel 21

4'-[(2-n-Propyl-4-methyl-6-(1-(1-n-propyl-n-butyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(1-n-propyl-n-butyl)-imidazol-4-yl)-benzimidazol-1-yl)-methyl]-biphenyl-2-carbonsäure-tert.butylester und Trifluoressigsäure in Methylenchlorid.
Ausbeute: 28,0 % der Theorie,
Schmelzpunkt: 236-238°C
$C_{35}H_{40}N_4O_2$ (548,73)
$R_f$-Wert: 0,61 (Kieselgel; Methylenchlorid/Methanol = 9:1)
Massenspektrum: m/e = 548

Beispiel 22

4'-[(2-Ethoxy-4-methyl-6-(1-cyclopropylmethyl-imidazol-4-yl)-benzimidazol-1-yl)-methyl]-biphenyl-2-carbonsäure

Hergestellt analog Beispiel A aus 4'-[(2-Ethoxy-4-methyl-6-(1-cyclopropylmethyl-imidazol-4-yl)-benzimidazol-1-yl)-methyl]-biphenyl-2-carbonsäure-tert.butylester und Trifluoressigsäure in Methylenchlorid.
Ausbeute: 52,0 % der Theorie,
Schmelzpunkt: 172-173°C
$C_{31}H_{30}N_4O_3$ (506,61)

| | | | | | | |
|---|---|---|---|---|---|---|
| Ber.: | C | 73,50 | H | 5,97 | N | 11,06 |
| Gef.: | | 73,36 | | 5,94 | | 11,30 |

$R_f$-Wert: 0,52 (Kieselgel; Methylenchlorid/Methanol = 9:1)
Massenspektrum: m/e = 506

Beispiel 23

4'-[(2-Ethoxy-4-methyl-6-(1-cyclopropylmethyl-imidazol-4-yl)-benzimidazol-1-yl)-methyl]-2-(1H-tetrazol-5-yl)-biphenyl

Hergestellt analog Beispiel 10 aus 4'-[(2-Ethoxy-4-methyl-6-(1-cyclopropylmethyl-imidazol-4-yl)-benzimidazol-1-yl)-methyl]-2-(2-triphenylmethyl-tetrazol-5-yl)-biphenyl.
Ausbeute: 42,0 % der Theorie,
Schmelzpunkt: amorph
$C_{31}H_{30}N_8O$ (530,64)
$R_f$-Wert: 0,50 (Kieselgel; Methylenchlorid/Methanol = 9:1)
Massenspektrum: m/e = 530

Beispiel 24

4'-[(2-n-Propyl-4-methyl-6-(1-(1-n-propyl-n-butyl)-imidazol-4-yl)-benzimidazol-1-yl)-methyl]-2-(1H-tetrazol-5-yl)-biphenyl

Hergestellt analog Beispiel 10 aus 4'-[(2-n-Propyl-4-methyl-6-(1-(1-n-propyl-n-butyl)-imidazol-4-yl)-benzimidazol-1-yl)-methyl]-2-(2-triphenylmethyl-tetrazol-5-yl)-biphenyl.
Ausbeute: 12,0 % der Theorie,
Schmelzpunkt: ab 150°C (sintern)
$C_{35}H_{40}N_8$ (572,76)
$R_f$-Wert: 0,34 (Kieselgel; Methylenchlorid/Methanol = 9:1)
Massenspektrum: m/e = 572

Beispiel 25

4'-[(2-n-Propyl-4-methyl-6-(1,2-dimethyl-imidazol-4-yl)-benzimidazol-1-yl)-methyl]-biphenyl-2-carbonsäure-hydrat

a) 4'-[(2-n-Propyl-4-methyl-6-(2-methyl-oxazol-4-yl)-benzimidazol-1-yl)-methyl]-biphenyl-2-carbonsäure-tert.butylester

Eine Lösung von 2,8 g (11 mMol) 2-n-Propyl-4-methyl-6-(2-methyl-oxazol-4-yl)-benzimidazol und 1,7 g (15 mMol) Kalium-tert.butylat in 60 ml Dimethylsulfoxid wird 15 Minuten bei Raumtemperatur gerührt. Dann werden 5,2 g (15 mMol) 4'-Brommethyl-biphenyl-2-carbonsäure-tert.butylester hinzugefügt und weitere 14 Stunden bei Raumtemperatur gerührt. Anschließend wird die Lösung in ca. 150 ml gesättigte Natriumchlorid-Lösung eingerührt, das ausgefallene Rohprodukt abgesaugt und durch Säulenchromatographie (400 g Kieselgel; Laufmittel: Methylenchlorid mit 1 bis 2 % Ethanol) gereinigt.
Ausbeute: 3,5 g (61,4 % der Theorie),
Schmelzpunkt: amorph
$R_f$-Wert: 0,90 (Kieselgel; Methylenchlorid/Ethanol = 4:1)

b) 4'-[(2-n-Propyl-4-methyl-6-(1,2-dimethyl-imidazol-4-yl)-benzimidazol-1-yl)-methyl]-biphenyl-2-carbonsäure-hydrat

Ein Gemisch aus 1,5 g (3 mMol) 4'-[(2-n-Propyl-4-methyl-6-(2-methyl-oxazol-4-yl)-benzimidazol-1-yl)-methyl]-biphenyl-2-carbonsäure-tert.butylester, 10 ml 40%iger N-Methylamin-Lösung und 15 ml N-Methylformamid wird zehn Stunden lang im Autoklaven auf 200°C erhitzt. Nach dem Abkühlen wird der Autoklaveninhalt mit ca. 40 ml Wasser verrührt, diese Suspension mit Eisessig auf pH 6,5 eingestellt, dann das ausgefallene Rohprodukt abgesaugt und in 1N Natronlauge gelöst. Diese Lösung wird nacheinander mit je 25 ml Essigester und Diethylether gewaschen, dann mit 20%iger Zitronensäure auf pH 6 eingestellt. Das ausgefallene Produkt wird abgesaugt, mit ca. 30 ml Wasser gewaschen und getrocknet, dann mit Diethylether verrieben und im Hochvakuum getrocknet.
Ausbeute: 950 mg (68 % der Theorie),
Schmelzpunkt: 239-240°C
$C_{30}H_{30}N_4O_2 \times H_2O$ (496,62)

| | | | | | | |
|---|---|---|---|---|---|---|
| Ber.: | C | 72,55 | H | 6,49 | N | 11,28 |
| Gef.: | | 72,62 | | 6,62 | | 11,54 |

$R_f$-Wert: 0,70 (Kieselgel; Methylenchlorid/Ethanol = 4:1)

Beispiel 26

4'-[(2-n-Propyl-4-methyl-6-(1,2-dimethyl-imidazol-4-yl)-benzimidazol-1-yl)-methyl]-2-(1H-tetrazol-5-yl)-biphenyl-hydrat

a) 4'-[(2-n-Propyl-4-methyl-6-(2-methyl-oxazol-4-yl)-benzimidazol-1-yl)-methyl]-2-(2-triphenylmethyl-tetrazol-5-yl)-biphenyl

Eine Lösung von 2,8 g (11 mMol) 2-n-Propyl-4-methyl-6-(2-methyl-oxazol-4-yl)-benzimidazol und 1,7 g (15 mMol) Kalium-tert.butylat in 60 ml Dimethylsulfoxid wird 15 Minuten bei Raumtemperatur gerührt. Dann werden 6,0 g (11 mMol) 4'-Brommethyl-2-(2-triphenylmethyl-tetrazol-5-yl)-biphenyl hinzugefügt und weitere drei Stunden bei Raumtemperatur gerührt. Anschließend wird die Lösung in ca. 150 ml gesättigte Natriumchlorid-Lösung eingerührt, das ausgefallene Rohprodukt abgesaugt und durch Säulenchromatographie (500 g Kieselgel; Laufmittel: Petrolether/Essigester = 1:1) gereinigt.
Ausbeute: 3,6 g (45 % der Theorie)

b) 4'-[(2-n-Propyl-4-methyl-6-(1,2-dimethyl-imidazol-4-yl)-benzimidazol-1-yl)-methyl]-2-(1H-tetrazol-5-yl)-biphenyl-hydrat

Ein Gemisch aus 3,6 g (4,9 mMol) 4'[(2-n-Propyl-4-methyl-6-(2-methyl-oxazol-4-yl)-benzimidazol-1-yl)-methyl]-2-(2-triphenylmethyl-tetrazol-5-yl)-biphenyl, 20 ml 40%iger N-Methylamin-Lösung und 30 ml N-Methylformamid wird zehn Stunden im Autoklaven auf 200°C erhitzt. Nach dem Abkühlen wird der Autoklaveninhalt mit ca. 50 ml Wasser verrührt, diese Suspension mit 20%iger Zitronensäure auf pH 6,5 eingestellt, dann das ausgefallene Rohprodukt abgesaugt und durch Säulenchromatographie (200 g Kieselgel; Laufmittel: Methylenchlorid mit 5 bis 20% Ethanol) gereinigt.
Ausbeute: 1,0 g (41 % der Theorie),
Schmelzpunkt: ab 195°C Sintern
$C_{30}H_{30}N_8$ x $H_2O$ (520,6)

| | | | | | | |
|---|---|---|---|---|---|---|
| Ber.: | C | 69,21 | H | 6,19 | N | 21,52 |
| Gef.: | | 68,99 | | 6,26 | | 21,37 |

Massensprektrum: m/e = 502

Beispiel 27

4'-[(2-Ethyl-4-methyl-6-(1-(2-methoxyethyl)-imidazol-4-yl)-benzimidazol-1-yl)-methyl]-biphenyl-2-carbonsäure

Hergestellt analog Beispiel A aus 4'-[(2-Ethyl-4-methyl-6-(1-(2-methoxyethyl)-imidazol-4-yl)-benzimidazol-1-yl)-methyl]-biphenyl-2-carbonsäure-tert.butylester und Trifluoressigsäure in Methylenchlorid.
Ausbeute: 49 % der Theorie,
Schmelzpunkt: 165-167°C
$C_{30}H_{30}N_4O_3$ (494,60)

| | | | | | | |
|---|---|---|---|---|---|---|
| Ber.: | C | 72,85 | H | 6,11 | N | 11,33 |
| Gef.: | | 72,62 | | 6,27 | | 11,35 |

Massensprektrum: m/e = 494

Beispiel 28

4'-[(2-Cyclopropyl-4-methyl-6-(1-(2-methoxyethyl)-imidazol-4-yl)-benzimidazol-1-yl)-methyl]-biphenyl-2-carbonsäure

Hergestellt analog Beispiel A aus 4'-[(2-Cyclopropyl-4-methyl-6-(1-(2-methoxyethyl)-imidazol-4-yl)-benzimidazol-1-yl)- methyl]-biphenyl-2-carbonsäure-tert.butylester und Trifluoressigsäure in Methylenchlorid.
Ausbeute: 78 % der Theorie,
Schmelzpunkt: 179-181°C
$C_{31}H_{30}N_4O_3$ (506,61)

| | | | | | | |
|---|---|---|---|---|---|---|
| Ber.: | C | 73,50 | H | 5,97 | N | 11,06 |
| Gef.: | | 73,37 | | 6,02 | | 11,02 |

Massensprektrum: m/e = 506

Beispiel 29

4'-[(2-n-Propyl-4-methyl-6-(1-aminocarbonylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-aminocarbonylmethyl-imidazol-4-yl)-benzimidazol-1-yl)-methyl]-biphenyl-2-carbonsäure-tert.butylester und Trifluoressigsäure in Methylenchlorid.
Ausbeute: 26 % der Theorie,
Schmelzpunkt: 190-192°C
$C_{30}H_{29}N_5O_3$ (507,60)
$R_f$-Wert: 0,44 (Kieselgel; Methylenchlorid/Methanol = 8:2)

Beispiel 30

4'-[(2-n-Propyl-4-methyl-6-(1-ethoxycarbonylmethyl-imidazol-4-yl)-benzimidazol-1-yl)-methyl]-biphenyl-2-carbonsäure

Hergestellt analog Beispiel A aus 4'-[(2-n-Propyl-4-methyl-6-(1-ethoxycarbonylmethyl-imidazol-4-yl)-benzimidazol-1-yl)-methyl]-biphenyl-2-carbonsäure-tert.butylester und Trifluoressigsäure in Methylenchlorid.
Ausbeute: 18 % der Theorie,
Schmelzpunkt: 223-224°C
$C_{32}H_{32}N_4O_4$ (536,63)
$R_f$-Wert: 0,69 (Kieselgel; Methylenchlorid/Methanol = 8:2)
Massensprektrum: m/e = 536

Beispiel 31

4'-[(2-Cyclopropyl-4-methyl-6-(1-(2-hydroxyethyl)-imidazol-4-yl)-benzimidazol-1-yl)-methyl]-biphenyl-2-carbonsäure

Eine Lösung von 500 mg (1,0 mMol) 4'-[(2-Cyclopropyl-4-methyl-6-(1-(2-methoxyethyl)-imidazol-4-yl)-benzimidazol-1-yl)-methyl]-biphenyl-2-carbonsäure und 1,5 g (6,0 mMol) Bortribromid in 50 ml Methylenchlorid wird 16 Stunden bei Raumtemperatur gerührt, dann mit ca. 30 ml Wasser versetzt und weitere zehn Minuten heftig gerührt. Dieses Gemisch wird bis zur Trockne eingeengt und der Rückstand in ca. 40 ml Ethanol zehn Minuten unter Rückfluß erhitzt. Wiederum wird bis zur Trockne eingedampft, der Rückstand in ca. 30 ml 2N Ammoniaklösung gelöst und diese Lösung mit 2N Essigsäure auf pH 5-6 eingestellt. Das dabei ausgefallene Rohprodukt wird abgesaugt und durch Säulenchromatographie (80 g Kieselgel; Laufmittel: Methylenchlorid/Methanol = 4:1) gereinigt.
Ausbeute: 150 mg (30 % der Theorie),
Schmelzpunkt: 220-222°C
$C_{30}H_{28}N_4O_3$ (492,58)
$R_f$-Wert: 0,20 (Kieselgel; Methylenchlorid/Methanol = 9:1)
Massensprektrum: m/e = 492

Beispiel 32

4'-[(2-n-Propyl-4-methyl-6-(1-(2-N-morpholinoethyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(2-N-morpholinoethyl)-imidazol-4-yl)-benzimidazol-1-yl)-methyl]-biphenyl-2-carbonsäure-tert.butylester und Trifluoressigsäure in Methylenchlorid.
Ausbeute: 54 % der Theorie,
Schmelzpunkt: 259-261°C
$C_{34}H_{37}N_5O_3$ (563,70)

| | | | | | | |
|---|---|---|---|---|---|---|
| Ber.: | C | 72,44 | H | 6,62 | N | 12,42 |
| Gef.: | | 72,68 | | 6,65 | | 12,53 |

Massensprektrum: m/e = 563

Beispiel 33

4'-[(2-n-Propyl-4-methyl-6-(1-(2-methoxyethoxy-2-ethyl)-imidazol-4-yl)-benzimidazol-1-yl)-methyl]-biphenyl-2-carbonsäure

Hergestellt analog Beispiel A aus 4'-[(2-n-Propyl-4-methyl-6-(1-(2-methoxyethoxy-2-ethyl)-imidazol-4-yl)-benzimidazol-1-yl)-methyl]-biphenyl-2-carbonsäure-tert.butylester und Trifluoressigsäure in Methylenchlorid.
Ausbeute: 49 % der Theorie,
Schmelzpunkt: 192-194°C
$C_{33}H_{36}N_4O_4$ (552,67)

| | | | | | | |
|---|---|---|---|---|---|---|
| Ber.: | C | 71,72 | H | 6,57 | N | 10,14 |
| Gef.: | | 71,52 | | 6,36 | | 10,25 |

$R_f$-Wert: 0,36 (Kieselgel; Dichlormethan/Methanol = 9:1)
Massensprektrum: m/e = 552

Beispiel 34

4'-[(2-n-Propyl-4-methyl-6-(1-(3-dimethylaminopropyl)-imidazol-4-yl)-benzimidazol-1-yl)-methyl]-biphenyl-2-carbonsäure-dihydrochlorid-pentahydrat

Hergestellt analog Beispiel A aus 4'-[(2-n-Propyl-4-methyl-6-(1-(3-dimethylaminopropyl)-imidazol-4-yl)-benzimidazol-1-yl)-methyl]-biphenyl-2-carbonsäure-tert.butylester und Trifluoressigsäure in Methylenchlorid.
Ausbeute: 12 % der Theorie,
Schmelzpunkt: ab 128°C (Zers.)
$C_{33}H_{37}N_5O_2$ x 2 HCl x 5 $H_2O$ (535,70)
$R_f$-Wert: 0,20 (Kieselgel; Dichlormethan/Methanol = 9:1)
Massensprektrum: m/e = 535

Beispiel 35

4'-[(2-n-Propyl-4-methyl-6-(2-methyl-thiazol-4-yl)-benzimidazol-1-yl)-methyl]-biphenyl-2-carbonsäure

Hergestellt analog Beispiel A aus 4'-[(2-n-Propyl-4-methyl-6-(2-methyl-thiazol-4-yl)-benzimidazol-1-yl)-methyl]-biphenyl-2-carbonsäure-tert.butylester und Trifluoressigsäure in Methylenchlorid.
Ausbeute: 32 % der Theorie,
Schmelzpunkt: 248-250°C
$C_{29}H_{27}N_3O_2S$ (481,62)

| | | | | | | |
|---|---|---|---|---|---|---|
| Ber.: | C | 72,32 | H | 5,65 | N | 8,72 |
| Gef.: | | 72,21 | | 5,83 | | 8,67 |

$R_f$-Wert: 0,26 (Kieselgel; Dichlormethan/Methanol = 9:1)
Massensprektrum: m/e = 481

Beispiel 36

4'-[(2-n-Propyl-4-methyl-6-(2-methyl-thiazol-4-yl)-benzimidazol-1-yl)-methyl]-2-(1H-tetrazol-5-yl)-biphenyl-dihydrochlorid

Hergestellt analog Beispiel 10 aus 4'-[(2-n-Propyl-4-methyl-6-(2-methyl-thiazol-4-yl)-benzimidazol-1-yl)-methyl]-2-(2-triphenylmethyl-tetrazol-5-yl)-biphenyl.
Ausbeute: 91 % der Theorie,
Schmelzpunkt: ab 219°C (Zers.)

$C_{29}H_{29}Cl_2N_7S$ (578,58)

| | | | | | | | | |
|---|---|---|---|---|---|---|---|---|
| Ber.: | C | 60,20 | H | 5,05 | N | 16,95 | Cl | 12,25 |
| Gef.: | | 59,96 | | 5,19 | | 16,63 | | 12,42 |

$R_f$-Wert: 0,32 (Kieselgel; Dichlormethan/Methanol = 9:1)
Massensprektrum: m/e = 505

Beispiel 37

4'-[(2-Ethyl-4-methyl-6-(1-(2-N-morpholinoethyl)-imidazol-4-yl)-benzimidazol-1-yl)-methyl]-biphenyl-2-carbonsäure

Hergestellt analog Beispiel A aus 4'-[(2-Ethyl-4-methyl-6-(1-(2-N-morpholinoethyl)-imidazol-4-yl)-benzimidazol-1-yl)-methyl]-biphenyl-2-carbonsäure-tert.butylester und Trifluoressigsäure in Methylenchlorid.
Ausbeute: 27 % der Theorie,
Schmelzpunkt: 201-202°C
$C_{33}H_{35}N_5O_3$ (549,65)

| | | | | | | |
|---|---|---|---|---|---|---|
| Ber.: | C | 72,11 | H | 6,42 | N | 12,74 |
| Gef.: | | 72,00 | | 6,48 | | 12,62 |

$R_f$-Wert: 0,36 (Kieselgel; Dichlormethan/Methanol = 9:1)
Massensprektrum: m/e = 549

Beispiel 38

4'-[(2-Ethyl-4-methyl-6-(1-(2-N-morpholinoethyl)-imidazol-4-yl)-benzimidazol-1-yl)-methyl]-2-(1H-tetrazol-5-yl)-biphenyl-hydrat

Hergestellt analog Beispiel 10 aus 4'-[(2-Ethyl-4-methyl-6-(1-(2-N-morpholinoethyl)-imidazol-4-yl)-benzimidazol-1-yl)-methyl]-2-(2-triphenylmethyl-tetrazol-5-yl)-biphenyl.
Ausbeute: 14 % der Theorie,
Schmelzpunkt: ab 180°C (Zers.)
$C_{33}H_{35}N_9O$ x $H_2O$ (573,68)

| | | | | | | |
|---|---|---|---|---|---|---|
| Ber.: | C | 66,98 | H | 6,30 | N | 21,31 |
| Gef.: | | 66,87 | | 6,36 | | 21,22 |

$R_f$-Wert: 0,31 (Kieselgel; Dichlormethan/Methanol = 9:1)
Massensprektrum: m/e = 573

Beispiel 39

4'-[(2-Ethyl-4-methyl-6-(1-(2-aminocarbonylethyl)-imidazol-4-yl)-benzimidazol-1-yl)-methyl]-biphenyl-2-carbonsäure

Hergestellt analog Beispiel A aus 4'-[(2-Ethyl-4-methyl-6-(1-(2-aminocarbonylethyl)-imidazol-4-yl)-benzimidazol-1-yl)-methyl]-biphenyl-2-carbonsäure-tert.butylester und Trifluoressigsäure in Methylenchlorid.
Ausbeute: 66 % der Theorie,
Schmelzpunkt: ab 185°C (Zers.)
$C_{30}H_{29}N_5O_3$ (507,59)

| | | | | | | |
|---|---|---|---|---|---|---|
| Ber.: | C | 70,99 | H | 5,76 | N | 13,80 |
| Gef.: | | 70,73 | | 5,72 | | 13,66 |

Massensprektrum: m/e = 507

Beispiel 40

4'-[(2-Ethyl-4-methyl-6-(1-(2-aminocarbonylethyl)-imidazol-4-yl)-benzimidazol-1-yl)-methyl]-2-(1H-tetrazol-5-yl)-biphenyl

Hergestellt analog Beispiel 10 aus 4'-[(2-Ethyl-4-methyl-6-(1-(2-aminocarbonylethyl)-imidazol-4-yl)-benzimidazol-1-yl)-methyl]-2-(2-triphenylmethyl-tetrazol-5-yl)-biphenyl.
Ausbeute: 42 % der Theorie,
Schmelzpunkt: ab 191°C (Zers.)
$C_{30}H_{29}N_9O$ (531,63)

| | | | | | | |
|---|---|---|---|---|---|---|
| Ber.: | C | 67,78 | H | 5,50 | N | 23,71 |
| Gef.: | | 67,79 | | 5,40 | | 23,66 |

$R_f$-Wert: 0,20 (Kieselgel; Dichlormethan/Methanol = 8:2)
Massensprektrum: m/e = 531

Beispiel 41

4'-[(2-Ethyl-4-methyl-6-(1-(2-N-pyrrolidinoethyl)-imidazol-4-yl)-benzimidazol-1-yl)-methyl]-biphenyl-2-carbonsäure

Hergestellt analog Beispiel A aus 4'-[(2-Ethyl-4-methyl-6-(1-(2-N-pyrrolidinoethyl)-imidazol-4-yl)-benzimidazol-1-yl)-methyl]-biphenyl-2-carbonsäure-tert.butylester und Trifluoressigsäure in Methylenchlorid.
Ausbeute: 60 % der Theorie,
Schmelzpunkt: 215-217°C
$C_{33}H_{35}N_5O_2$ (533,67)

| | | | | | | |
|---|---|---|---|---|---|---|
| Ber.: | C | 74,27 | H | 6,61 | N | 13,12 |
| Gef.: | | 74,03 | | 6,85 | | 13,11 |

$R_f$-Wert: 0,30 (Kieselgel; Dichlormethan/Methanol = 8:2)
Massensprektrum: m/e = 533

Beispiel 42

4'-[(2-Ethyl-4-methyl-6-(1-(2-N-pyrrolidinoethyl)-imidazol-4-yl)-benzimidazol-1-yl)-methyl]-2-(1H-tetrazol-5-yl)-biphenyl

Hergestellt analog Beispiel 10 aus 4'-[(2-Ethyl-4-methyl-6-(1-(2-N-pyrrolidinoethyl)-imidazol-4-yl)-benzimidazol-1-yl)-methyl]-2-(2-triphenylmethyl-tetrazol-5-yl)-biphenyl.
Ausbeute: 38 % der Theorie,
Schmelzpunkt: ab 128°C (Sintern)
$C_{33}H_{35}N_9$ (551,71)

| | | | | | | |
|---|---|---|---|---|---|---|
| Ber.: | C | 71,84 | H | 6,39 | N | 22,85 |
| Gef.: | | 71,63 | | 6,20 | | 22,49 |

$R_f$-Wert: 0,23 (Kieselgel; Dichlormethan/Methanol = 8:2)

Beispiel 43

4'-[(2-Ethyl-4-methyl-6-(1-(2-diethylaminoethyl)-imidazol-4-yl)-benzimidazol-1-yl)-methyl]-biphenyl-2-carbonsäure-dihydrochlorid

Hergestellt analog Beispiel A aus 4'-[(2-Ethyl-4-methyl-6-(1-(2-diethylaminoethyl)-imidazol-4-yl)-benzimidazol-1-yl)-methyl]-biphenyl-2-carbonsäure-tert.butylester und Trifluoressigsäure in Methylenchlorid.
Ausbeute: 32 % der Theorie,
Schmelzpunkt: 255-257°C (Zers.)
$C_{33}H_{37}N_5O_2$ x 2 HCL (608,60)
$R_f$-Wert: 0,24 (Kieselgel; Dichlormethan/Methanol = 9:1)
Massenspektrum: m/e = 535

Beispiel 44

4'-[(2-Ethyl-4-methyl-6-(1-(2-diethylaminoethyl)-imidazol-4-yl)-benzimidazol-1-yl)-methyl]-2-(1H-tetrazol-5-yl)-biphenyl

Hergestellt analog Beispiel 10 aus 4'-[(2-Ethyl-4-methyl-6-(1-(2-diethylaminoethyl)-imidazol-4-yl)-benzimidazol-1-yl)-methyl]-2-(2-triphenylmethyl-tetrazol-5-yl)-biphenyl.
Ausbeute: 51 % der Theorie,
Schmelzpunkt: 191-193°C
$C_{33}H_{37}N_9$ (559,70)

| | | | | | | |
|---|---|---|---|---|---|---|
| Ber.: | C | 70,81 | H | 6,66 | N | 22,52 |
| Gef.: | | 70,59 | | 6,66 | | 22,58 |

$R_f$-Wert: 0,30 (Kieselgel; Dichlormethan/Methanol = 8:2)

Beispiel 45

4'-[(2-Ethyl-4-methyl-6-(1-(3-N-piperidinopropyl)-imidazol-4-yl)-benzimidazol-1-yl)-methyl]-biphenyl-2-carbonsäure

Hergestellt analog Beispiel A aus 4'-[(2-Ethyl-4-methyl-6-( 1-(3-N-piperidinopropyl)-imidazol-4-yl)-benzimidazol-1-yl)-methyl]-biphenyl-2-carbonsäure-tert.butylester und Trifluoressigsäure in Methylenchlorid.
Ausbeute: 19 % der Theorie,
Schmelzpunkt: amorph
$C_{35}H_{39}N_5O_2$ (561,73)

| | | | | | | |
|---|---|---|---|---|---|---|
| Ber.: | C | 74,84 | H | 7,00 | N | 12,47 |
| Gef.: | | 74,61 | | 6,92 | | 12,31 |

$R_f$-Wert: 0,34 (Kieselgel; Dichlormethan/Methanol = 8:2)

Beispiel 46

4'-[(2-Ethyl-4-methyl-6-(1-(3-N-piperidinopropyl)-imidazol-4-yl)-benzimidazol-1-yl)-methyl]-2-(1H-tetrazol-5-yl)-biphenyl

Hergestellt analog Beispiel 10 aus 4'-[(2-Ethyl-4-methyl-6-(1-(3-N-piperidinopropyl)-imidazol-4-yl)-benzimidazol-1-yl)-methyl]-2-(2-triphenylmethyl-tetrazol-5-yl)-biphenyl.
Ausbeute: 71 % der Theorie,
Schmelzpunkt: ab 140°C (Zers.)
$C_{35}H_{39}N_9$ (585,76)

| | | | | | | |
|---|---|---|---|---|---|---|
| Ber.: | C | 71,77 | H | 6,71 | N | 21,52 |
| Gef.: | | 71,58 | | 6,68 | | 21,44 |

$R_f$-Wert: 0,22 (Kieselgel; Dichlormethan/Methanol = 8:2)

Bei den nachfolgenden pharmazeutischen Anwendungsbeispielen kann als Wirksubstanz jede geeignete Verbindung der Formel I, insbesondere diejenigen, in denen $R_4$ eine Carboxy- oder 1H-Tetrazolylgruppe darstellt, eingesetzt werden:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50°C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70°C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

**Patentansprüche**

1. Benzimidazole der allgemeinen Formel

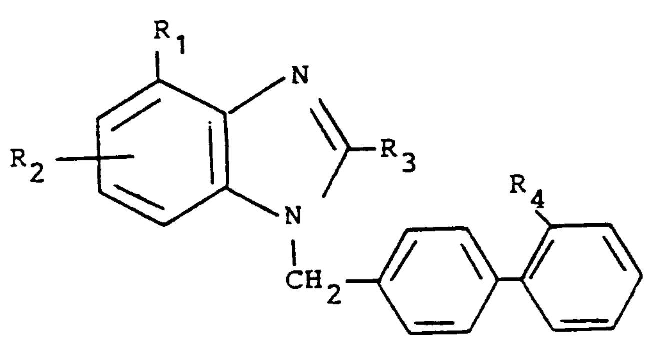

, (I)

in der
$R_1$ eine Alkylgruppe mit 1 bis 3 Kohlenstoffatomen, ein Wasserstoff-, Fluor-, Chlor- oder Bromatom,

$R_2$ eine in 2-Stellung durch eine Phenylgruppe substituierte Oxazol-4-yl- oder Thiazol-4-yl-Gruppe oder eine gegebenenfalls in 2-Stellung durch eine Alkylgruppe mit 1 bis 6 Kohlenstoffatomen oder durch eine Phenylgruppe substituierte Imidazol-4-yl-Gruppe, wobei die Imidazol-4-yl-Gruppe in 1-Stellung durch eine Alkylgruppe mit 1 bis 7 Kohlenstoffatomen, die in 1-, 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Alkoxy-, Alkoxyalkoxy-, Dialkylamino-, Pyrrolidino-, Piperidino-, Hexamethylenimino-, Morpholino-, Thiomorpholino-, 1-Oxido-thiomorpholino- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine durch Fluor- oder Chloratome, durch Trifluormethyl-, Methyl- oder Methoxygruppen mono- oder disubstituierte Phenylgruppe substituiert ist, oder durch eine durch zwei Phenylgruppen substituierte Alkylgruppe substituiert ist, wobei, soweit nichts anderes erwähnt wurde, die vorstehend erwähnten Alkyl- und Alkoxyteile jeweils 1 bis 3 Kohlenstoffatome enthalten können,
$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und
$R_4$ eine in vivo in eine Carboxygruppe überführbare Gruppe, eine Carboxy-, Cyano-, 1H-Tetrazolyl-, 1-Triphenylmethyl-tetrazolyl- oder 2-Triphenylmethyl-tetrazolylgruppe bedeuten,
sowie die Verbindungen

a) 4'-[(2-n-Propyl-4-methyl-6-(1-(2-phenylethyl)-imidazol-4-yl)-benzimidazol-1-yl)-methyl]-biphenyl-2-carbonsäure,

b) 4'-[(2-n-Propyl-4-methyl-6-(2-methyl-oxazol-4-yl)-benzimidazol-1-yl)-methyl]-biphenyl-2-carbonsäure,

c) 4'-[(2-n-Propyl-4-methyl-6-(2-methyl-oxazol-4-yl)-benzimidazol-1-yl)-methyl]-2-(1H-tetrazol-5-yl)-biphenyl,

d) 4'-[(2-Ethoxy-6-(1-isopropyl-imidazol-4-yl)-benzimidazol-1-yl)-methyl]-2-(1H-tetrazol-5-yl)-biphenyl,

e) 4'-[(2-Ethoxy-6-(1-isopropyl-imidazol-4-yl)-benzimidazol-1-yl)-methyl]-biphenyl-2-carbonsäure,

f) 4'-[(2-Ethoxy-5-(1-isopropyl-imidazol-4-yl)-benzimidazol-1-yl)-methyl]-2-(1H-tetrazol-5-yl)-biphenyl,

g) 4'-[(2-n-Propyl-4-methyl-6-(1-cycloheptyl-imidazol-4-yl)-benzimidazol-1-yl)-methyl]-biphenyl-2-carbonsäure,

h) 4'-[(2-n-Propyl-4-methyl-6-(1-cycloheptyl-imidazol-4-yl)-benzimidazol-1-yl)-methyl]-2-(1H-tetrazol-5-yl)-biphenyl,

i) 4'-[(2-n-Propyl-4-methyl-6-(1-(1-n-propyl-n-butyl)-imidazol-4-yl)-benzimidazol-1-yl)-methyl]-2-(1H-tetrazol-5-yl)-biphenyl,

j) 4'-[(2-n-Propyl-4-methyl-6-(1,2-dimethyl-imidazol-4-yl)-benzimidazol-1-yl)-methyl]-biphenyl-2-carbonsäure,

k) 4'-[(2-n-Propyl-4-methyl-6-(1,2-dimethyl-imidazol-4-yl)-benzimidazol-1-yl)-methyl]-2-(1H-tetrazol-5-yl)-biphenyl,

l) 4'-[(2-n-Propyl-4-methyl-6-(2-methyl-thiazol-4-yl)-benzimidazol-1-yl)-methyl]-biphenyl-2-carbonsäure und

m) 4'-[(2-n-Propyl-4-methyl-6-(2-methyl-thiazol-4-yl)-benzimidazol-1-yl)-methyl]-2-(1H-tetrazol-5-yl)-biphenyl,

und deren Salze.

**2.** Benzimidazole der allgemeinen Formel I gemäß Anspruch 1, in denen
$R_1$ eine Alkylgruppe mit 1 bis 3 Kohlenstoffatomen, ein Wasserstoff-, Fluor-, Chlor- oder Bromatom,
$R_2$ in 6-Stellung eine in 2-Stellung durch eine Phenylgruppe substituierte Oxazol-4-yl- oder Thiazol-4-yl-Gruppe oder
eine gegebenenfalls in 2-Stellung durch eine Alkylgruppe mit 1 bis 6 Kohlenstoffatomen oder durch eine Phenylgruppe substituierte Imidazol-4-yl-Gruppe, wobei die Imidazol-4-yl-Gruppe in 1-Stellung durch eine Alkylgruppe mit 1 bis 7 Kohlenstoffatomen, die in 1-, 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Alkoxy-, Alkoxyalkoxy-, Dialkylamino-, Pyrrolidino-, Piperidino-, Hexamethylenimino-, Morpholino-, Thiomorpholino-, 1-Oxido-thiomorpholino- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine durch Fluor- oder Chloratome, durch Trifluormethyl-, Methyl- oder Methoxygruppen mono- oder disubstituierte Phenylgruppe substituiert ist, oder durch eine durch zwei Phenylgruppen substituierte Alkylgruppe substituiert ist, wobei, soweit nichts anderes erwähnt wurde, die vorstehend erwähnten Alkyl- und Alkoxyteile jeweils 1 bis 3 Kohlenstoffatome enthalten können,
$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und
$R_4$ eine in vivo in eine Carboxygruppe überführbare Gruppe, eine Carboxy-, Cyano-, 1H-Tetrazolyl-, 1-Triphenylmethyl-tetrazolyl- oder 2-Triphenylmethyl-tetrazolylgruppe bedeuten,
und deren Salze.

**3.** Benzimidazole der allgemeinen Formel I gemäß Anspruch 1, in der
$R_1$ ein Wasserstoff- oder Chloratom oder eine Methylgruppe,
$R_2$ in 6-Stellung eine in 2-Stellung durch eine Phenylgruppe substituierte Oxazol-4-yl- oder Thiazol-4-yl-Gruppe oder eine gegebenenfalls in 2-Stellung durch eine Methylgruppe substituierte Imidazol-4-yl-Gruppe, die in 1-Stellung durch eine Alkylgruppe mit 1 bis 7 Kohlenstoffatomen, die in 1-, 2-, 3-, 4-, 5-, 6- oder 7-Stellung durch eine Carboxy-, Methoxycarbonyl-, Aminocarbonyl-, Methylaminocarbonyl-, Ethylaminocarbonyl-, n-Propylaminocarbonyl-, Dimethylaminocarbonyl-, Morpholinocarbonyl-, Thiomorpholinocarbonyl- oder 1-Oxido-thiomorpholinocarbonylgruppe substituiert ist, durch eine Alkylgruppe mit 2 bis 4 Kohlenstoffatomen, die in 2-, 3- oder 4-Stellung durch eine Hydroxy-, Methoxy-, 2-Methoxyethoxy-, Dimethylamino-, Diethylamino-, Pyrrolidino-, Piperidino-, Morpholino- oder Imidazol-1-yl-Gruppe substituiert ist, durch eine 2,2,2-Trifluorethyl-, 3,3,3-Trifluorpropyl-, Cyclopropylmethyl-, Cyclobutylmethyl-, Cyclopentylmethyl-, Cyclohexylmethyl-, 4-Fluorbenzyl-, 3-Chlorbenzyl-, 4-Methylbenzyl-, 4-Trifluormethylbenzyl-, 3,5-Dimethoxybenzyl- oder 2,2-Diphenylethylgruppe substituiert ist,
$R_3$ eine Alkylgruppe mit 2 bis 4 Kohlenstoffatomen, eine Alkoxy- oder Alkylthiogruppe mit jeweils 2 oder 3 Kohlenstoffatomen im Alkylteil, eine Cyclopropyl- oder Cyclobutylgruppe und
$R_4$ eine in vivo in eine Carboxygruppe überführbare Gruppe, eine Carboxy- oder 1H-Tetrazolylgruppe bedeuten,
und deren Salze.

**4.** Benzimidazole der allgemeinen Formel I gemäß Anspruch 1, in der
$R_1$, $R_2$ und $R_3$ wie in den Ansprüchen 1 bis 3 definiert sind und
$R_4$ eine Carboxygruppe oder eine Gruppe der Formeln

$$- CO - OR',$$

$$- CO - O - (HCR'') - O - CO - R''' \text{ und}$$

$$- CO - O - (HCR'') - O - CO - OR''' \text{ darstellt,}$$

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten,
und deren Salze.

**5.** Benzimidazole der allgemeinen Formel I gemäß Anspruch 1, in der
$R_1$ ein Wasserstoffatom oder eine Methylgruppe,
$R_2$ in 6-Stellung eine in 2-Stellung durch eine Phenylgruppe substituierte Oxazol-4-yl- oder Thiazol-4-yl-Gruppe oder
eine gegebenenfalls in 2-Stellung durch eine Methylgruppe substituierte Imidazol-4-yl-Gruppe, die in 1-Stellung durch eine Alkylgruppe mit 1 bis 7 Kohlenstoffatomen, die in 1-, 2-, 3-, 4-, 5-, 6- oder 7-Stellung durch eine Carboxy-, Methoxycarbonyl-, Dimethylaminocarbonyl- oder Morpholinocarbonylgruppe substituiert ist, durch eine Alkylgruppe mit 2 bis 4 Kohlenstoffatomen, die in 2-, 3- oder 4-Stellung durch eine Hydroxy-, Methoxy-, 2-Methoxyethoxy-, Dimethylamino-, Diethylamino-, Pyrrolidino-, Piperidino-, Morpholino- oder Imidazol-1-yl-Gruppe substituiert ist, durch eine 2,2,2-Trifluorethyl-, 3,3,3-Trifluorpropyl-, Cyclopropylmethyl-, Cyclobutylmethyl-, Cyclopentylmethyl-, Cyclohexylmethyl-, oder 4-Fluorbenzylgruppe substituiert ist,
$R_3$ eine Alkylgruppe mit 2 bis 4 Kohlenstoffatomen, eine Ethoxy-, Ethylthio-, Cyclopropyl- oder Cyclobutylgruppe und
$R_4$ eine Carboxy- oder 1H-Tetrazolylgruppe bedeuten,
und deren Salze.

**6.** Folgende Verbindungen der allgemeinen Formel I gemäß Anspruch 1:

(i) 4'-[(2-n-Propyl-4-methyl-6-(1-(2-N-morpholinoethyl)-imidazol-4-yl)-benzimidazol-1-yl)-methyl]-biphenyl-2-carbonsäure,

(ii) 4'-[(2-n-Propyl-4-methyl-6-(1-(2-methoxyethoxy-2-ethyl)-imidazol-4-yl)-benzimidazol-1-yl)-methyl]-biphenyl-2-carbonsäure,

(iii) 4'-[(2-Ethyl-4-methyl-6-(1-(2-diethylaminoethyl)-imidazol-4-yl)-benzimidazol-1-yl)-methyl]-2-(1H-tetrazol-5-yl)-biphenyl,

(iv) 4'-[(2-Ethyl-4-methyl-6-(1-(3-N-piperidinopropyl)-imidazol-4-yl)-benzimidazol-1-yl)-methyl]-2-(1H-tetrazol-5-yl)-biphenyl,

(v) 4'-[(2-n-Propyl-4-methyl-6-(1-(3-dimethylaminopropyl)-imidazol-4-yl)-benzimidazol-1-yl)-methyl]-biphenyl-2-carbonsäure,

(vi) 4'-[(2-Ethyl-4-methyl-6-(1-(2-N-morpholinoethyl)-imidazol-4-yl)-benzimidazol-1-yl)-methyl]-biphenyl-2-carbonsäure,

(vii) 4'-[(2-Ethyl-4-methyl-6-(1-(2-N-morpholinoethyl)-imidazol-4-yl)-benzimidazol-1-yl)-methyl]-2-(1H-tetrazol-5-yl)-biphenyl,

(viii) 4'-[(2-Ethyl-4-methyl-6-(1-(2-N-pyrrolidinoethyl)-imidazol-4-yl)-benzimidazol-1-yl)-methyl]-biphenyl-2-carbonsäure,

(ix) 4'-[(2-Ethyl-4-methyl-6-(1-(2-N-pyrrolidinoethyl)-imidazol-4-yl)-benzimidazol-1-yl)-methyl]-2-(1H-tetrazol-5-yl)-biphenyl,

(x) 4'-[(2-Ethyl-4-methyl-6-(1-(2-diethylaminoethyl)-imidazol-4-yl)-benzimidazol-1-yl)-methyl]-biphenyl-2-carbonsäure,

(xi) 4'-[(2-Ethyl-4-methyl-6-(1-(3-N-piperidinopropyl)-imidazol-4-yl)-benzimidazol-1-yl)-methyl]-biphenyl-2-carbonsäure

und deren Salze.

**7.** 4'-[(2-n-Propyl-4-methyl-6-(1-(2-N-morpholinoethyl)-imidazol-4-yl)-benzimidazol-1-yl)-methyl]-biphenyl-2-carbonsäure und deren Salze.

**8.** Physiologisch verträgliche Salze der Verbindungen nach mindestens einem der Ansprüche 1 bis 7 mit anorganischen oder organischen Säuren oder Basen.

**9.** Arzneimittel, enthaltend eine Verbindung nach mindestens einem der Ansprüche 1 bis 7 oder ein physiologisch verträgliches Salz gemäß Anspruch 8 neben gegebenenfalls einem oder mehreren inerten Trägerstoffen und/oder Verdünnungsmitteln.

**10.** Verwendung einer Verbindung nach mindestens einem der Ansprüche 1 bis 8 zur Herstellung eines Arzneimittels mit Angiotensin-antagonistischer Wirkung.

**11.** Verfahren zur Herstellung eines Arzneimittels gemäß Anspruch 9, dadurch gekennzeichnet, daß auf nichtchemischem Wege eine Verbindung nach mindestens einem der Ansprüche 1 bis 8 in einen oder mehrere inerte Trägerstoffe und/oder Verdünnungsmittel eingearbeitet wird.

**12.** Verfahren zur Herstellung der Benzimidazole gemäß den Ansprüchen 1 bis 8, dadurch gekennzeichnet, daß

a) eine gegebenenfalls im Reaktionsgemisch gebildete Verbindung der allgemeinen Formel

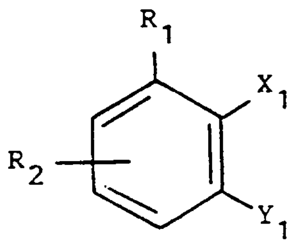

,(II)

in der
$R_1$ und $R_2$ wie in den Ansprüchen 1 bis 7 definiert sind, einer der Reste $X_1$ oder $Y_1$ eine Gruppe der allgemeinen Formel

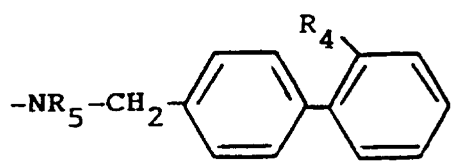

und der andere der Reste $X_1$ oder $Y_1$ eine Gruppe der allgemeinen Formel

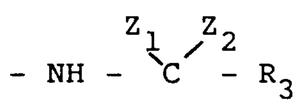

darstellen, wobei
$R_3$ und $R_4$ wie in den Ansprüchen 1 bis 7 definiert sind,
$R_5$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch Alkylgruppen mit 1 bis 6 Kohlenstoffatomen substituierte Hydroxy- oder Mercaptogruppen oder $Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und ein gegebenenfalls so erhaltenes N-Oxid reduziert wird oder

b) ein Benzimidazol der allgemeinen Formel

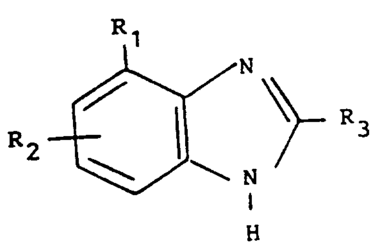

,(III)

in der

$R_1$ bis $R_3$ wie in den Ansprüchen 1 bis 7 definiert sind, mit einer Biphenylverbindung der allgemeinen Formel

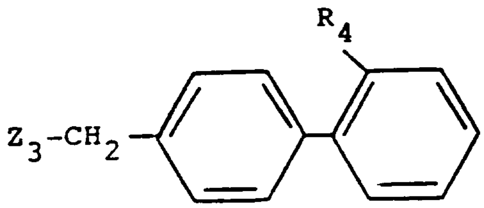

, (IV)

in der
$R_4$ wie in den Ansprüchen 1 bis 7 definiert ist und
$Z_3$ eine nukleophile Austrittsgruppe darstellt, umgesetzt wird oder

c) zur Herstellung einer Verbindung der allgemeinen Formel I, in der $R_4$ eine Carboxygruppe darstellt, eine Verbindung der allgemeinen Formel

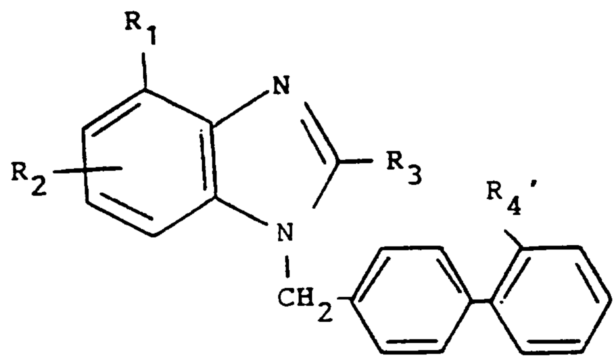

, (V)

in der
$R_1$ bis $R_3$ wie in den Ansprüchen 1 bis 7 definiert sind und $R_4$' eine mittels Hydrolyse, Thermolyse oder Hydrogenolyse in eine Carboxygruppe überführbare Gruppe darstellt, in eine entsprechende Carboxyverbindung übergeführt wird oder

d) zur Herstellung einer Verbindung der allgemeinen Formel I, in der $R_4$ eine 1H-Tetrazolylgruppe darstellt, ein Schutzrest von einer Verbindung der allgemeinen Formel

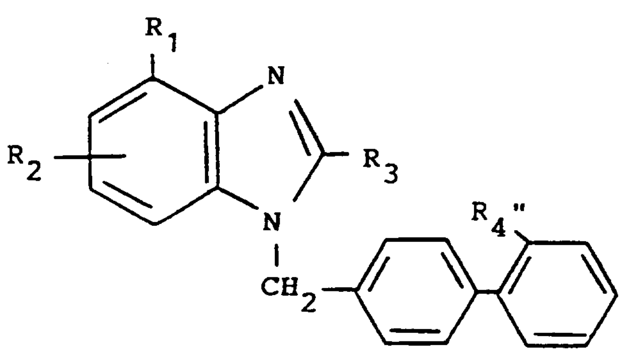

, (VI)

in der
$R_1$, $R_2$ und $R_3$ wie in den Ansprüchen 1 bis 7 definiert sind und
$R_4$" eine in 1- oder 3-Stellung durch einen Schutzrest geschützte 1H-Tetrazolylgruppe darstellt, abgespalten wird oder

e) zur Herstellung einer Verbindung der allgemeinen Formel I, in der $R_4$ eine 1H-Tetrazolylgruppe darstellt, eine Verbindung der allgemeinen Formel

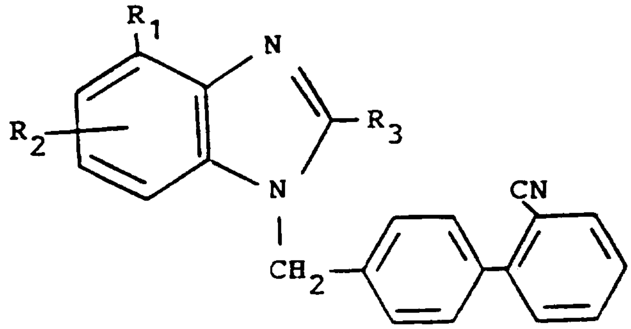

,(VII)

in der

$R_1$ bis $R_3$ wie in den Ansprüchen 1 bis 7 definiert sind, mit Stickstoffwasserstoffsäure oder deren Salzen umgesetzt wird und

gewünschtenfalls anschließend eine so erhaltene Verbindung der allgemeinen Formel I, in der $R_4$ eine Carboxygruppe darstellt, mittels Veresterung in eine entsprechende Verbindung der allgemeinen Formel I, in der $R_4$ eine in vivo in eine Carboxygruppe überführbare Gruppe darstellt, übergeführt wird und/oder

eine so erhaltene Verbindung der allgemeinen Formel I, in der $R_2$ eine Imidazol-4-yl-gruppe darstellt, die in 1-Stellung durch eine Alkylgruppe mit 2 bis 4 Kohlenstoffatomen substituiert ist, wobei die Alkylgruppe in 2-, 3- oder 4-Stellung durch eine Alkoxy- oder Alkoxyalkoxygruppe substituiert ist, mittels Etherspaltung in eine entsprechende Verbindung der allgemeinen Formel I übergeführt wird, in der $R_2$ eine Imidazol-4-yl-gruppe darstellt, die in 1-Stellung durch eine Alkylgruppe mit 2 bis 4 Kohlenstoffatomen substituiert ist, wobei die Alkylgruppe in 2-, 3- oder 4-Stellung durch eine Hydroxygruppe substituiert ist, übergeführt wird und

erforderlichenfalls ein während der Umsetzungen a) bis e) zum Schutze von reaktiven Gruppen verwendeter Schutzrest abgespalten wird und/oder

anschließend von einem so erhaltenen 1-, 3-Isomerengemisch einer Verbindung der allgemeinen Formel I mittels Isomerentrennung das 1-Isomere abgetrennt wird oder

eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

## Claims

1. Benzimidazoles of general formula

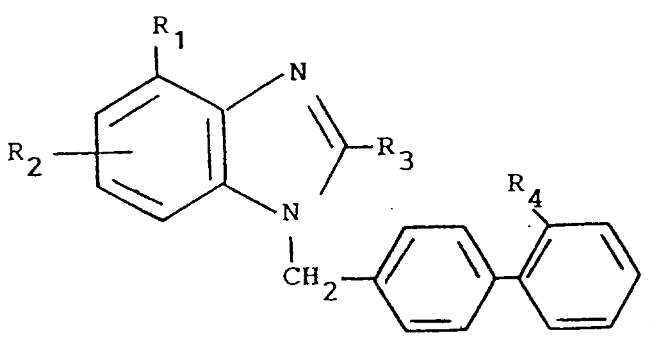

(I)

wherein

$R_1$ represents a $C_{1-3}$-alkyl group, a hydrogen, fluorine, chlorine or bromine atom,

$R_2$ represents an oxazol-4-yl or thiazol-4-yl group substituted in the 2-position by a phenyl group, or an imidazol-4-yl group optionally substituted in the 2-position by a $C_{1-6}$-alkyl group or by a phenyl group, whilst the imidazol-4-yl group is substituted in the 1-position

by a $C_{1-7}$-alkyl group which may be substituted in the 1-, 2-, 3-, 4-, 5-, 6- or 7-position by a carboxy, alkoxycarbonyl,

aminocarbonyl, alkylaminocarbonyl, dialkylaminocarbonyl, morpholinocarbonyl, thiomorpholinocarbonyl or 1-oxido-thiomorpholinocarbonyl group,
by a $C_{2-4}$-alkyl group substituted in the 2-, 3- or 4-position by a hydroxy, alkoxy, alkoxyalkoxy, dialkylamino, pyrrolidino, piperidino, hexamethyleneimino, morpholino, thiomorpholino, 1-oxido-thiomorpholino or imidazol-1-yl group,
by an alkyl group which is substituted by a trifluoromethyl group, by a $C_{3-7}$-cycloalkyl group or by a phenyl group optionally mono- or disubstituted by fluorine or chlorine atoms, or by trifluoromethyl, methyl or methoxy groups,
or by an alkyl group substituted by two phenyl groups
where unless otherwise specified the above-mentioned alkyl and alkoxy moieties may each contain 1 to 3 carbon atoms,
$R_3$ represents a $C_{2-4}$-alkyl group, an alkoxy or alkylthio group each having 2 or 3 carbon atoms in the alkyl moiety, a cyclopropyl or cyclobutyl group and
$R_4$ represents a group which may be converted in vivo into a carboxy group, or a carboxy, cyano, 1H-tetrazolyl, 1-triphenylmethyl-tetrazolyl group or 2-triphenylmethyl-tetrazolyl group,
and the compounds

a) 4'-[(2-n-propyl-4-methyl-6-(1-(2-phenylethyl)-imidazol-4-yl)-benzimidazol-1-yl)-methyl]-biphenyl-2-carboxylic acid,

b) 4'-[(2-n-propyl-4-methyl-6-(2-methyl-oxazol-4-yl)-benzimidazol-1-yl)-methyl]-biphenyl-2-carboxylic acid,

c) 4'-[(2-n-propyl-4-methyl-6-(2-methyl-oxazol-4-yl)-benzimidazol-1-yl)-methyl]-2-(1H-tetrazol-5-yl)-biphenyl,

d) 4'-[(2-ethoxy-6-(1-isopropyl-imidazol-4-yl)-benzimidazol-1-yl)-methyl]-2-(1H-tetrazol-5-yl)- biphenyl,

e) 4'-[(2-ethoxy-6-(1-isopropyl-imidazol-4-yl)-benzimidazol-1-yl)-methyl]-biphenyl-2-carboxylic acid,

f) 4'-[(2-ethoxy-5-(1-isopropyl-imidazol-4-yl)-benzimidazol-1-yl)-methyl]-2-(1H-tetrazol-5-yl)-biphenyl,

g) 4'-[(2-n-propyl-4-methyl-6-(1-cycloheptyl-imidazol-4-yl)-benzimidazol-1-yl)-methyl]-biphenyl-2-carboxylic acid,

h) 4'-[(2-n-propyl-4-methyl-6-(1-cycloheptyl-imidazol-4-yl)-benzimidazol-1-yl)-methyl]-2-(1H-tetrazol-5-yl)-biphenyl,

i) 4'-[(2-n-propyl-4-methyl-6-(1-(1-n-propyl-n-butyl)-imidazol-4-yl)-benzimidazol-1-yl)-methyl]-2-(1H-tetrazol-5-yl)-biphenyl,

j) 4'-[(2-n-propyl-4-methyl-6-(1,2-dimethyl-imidazol-4-yl)-benzimidazol-1-yl)-methyl]-biphenyl-2-carboxylic acid,

k) 4'-[(2-n-propyl-4-methyl-6-(1,2-dimethyl-imidazol-4-yl)-benzimidazol-1-yl)-methyl]-2-(1H-tetrazol-5-yl)-biphenyl,

l) 4'-[(2-n-propyl-4-methyl-6-(2-methyl-thiazol-4-yl)-benzimidazol-1-yl)-methyl]-biphenyl-2-carboxylic acid and

m) 4'-[(2-n-propyl-4-methyl-6-(2-methyl-thiazol-4-yl)-benzimidazol-1-yl)-methyl]-2-(1H-tetrazol-5-yl)-biphenyl,

and the salts thereof.

**2.** Benzimidazoles of general formula I according to claim 1, wherein
$R_1$ represents a $C_{1-3}$-alkyl group, a hydrogen, fluorine, chlorine or bromine atom,
$R_2$ in the 6 position represents an oxazol-4-yl or thiazol-4-yl group optionally substituted by a phenyl group in the 2-position, or an imidazol-4-yl group optionally substituted in the 2-position by a $C_{1-6}$-alkyl or phenyl group, wherein the imidazol-4-yl group may be substituted in the 1-position
by a $C_{1-7}$-alkyl group, which itself may be substituted in the 1-, 2-, 3-, 4-, 5-, 6- or 7-position by a carboxy, alkoxycarbonyl, aminocarbonyl, alkylaminocarbonyl, dialkylaminocarbonyl, morpholinocarbonyl, thiomorpholinocarbonyl or 1-oxido-thiomorpholinocarbonyl group,
by a $C_{2-4}$-alkyl group which is substituted in the 2-, 3-or 4-position by a hydroxy, alkoxy, alkoxyalkoxy, dialkylamino, pyrrolidino, piperidino, hexamethyleneimino, morpholino, thiomorpholino, 1-oxido-thiomorpholino or imidazol-1-yl

group, by an alkyl group substituted by a trifluoromethyl group, by a $C_{3-7}$-cycloalkyl group,
$R_3$ represents a $C_{2-4}$-alkyl group, an alkoxy or alkylthio group each having 2 or 3 carbon atoms in the alkyl moiety, a cyclopropyl or cyclobutyl group and
$R_4$ represents a group which may be converted into a carboxy group in vivo, or a carboxy, cyano, 1H-tetrazolyl, 1-triphenylmethyl-tetrazolyl or 2-triphenylmethyl-tetrazolyl group,
and the salts thereof.

**3.** Benzimidazoles of general formula I according to claim 1, wherein
$R_1$ denotes a hydrogen or chlorine atom or a methyl group,
$R_2$ in the 6-position represents an oxazol-4-yl or thiazol-4-yl substituted in the 2-position by a phenyl group or an imidazol-4-yl optionally substituted in the 2-position by a methyl group, which is substituted in the 1-position by a $C_{1-7}$-alkyl group which is substituted in the 1-, 2-, 3-, 4-, 5-, 6- or 7-position by a carboxy, methoxycarbonyl, aminocarbonyl, methylaminocarbonyl, ethylaminocarbonyl, n-propylaminocarbonyl, dimethylaminocarbonyl, morpholinocarbonyl, thiomorpholinocarbonyl or 1-oxido-thiomorpholinocarbonyl group, by a $C_{2-4}$-alkyl group which is substituted in the 2-, 3- or 4-position by a hydroxy, methoxy, 2-methoxyethoxy, dimethylamino, diethylamino, pyrrolidino, piperidino, morpholino or imidazol-1-yl group, or by a 2,2,2-trifluoroethyl, 3,3,3-trifluoropropyl, cyclopropylmethyl, cyclobutylmethyl, cyclopentylmethyl, cyclohexylmethyl, 4-fluorobenzyl, 3-chlorobenzyl, 4-methylbenzyl, 4-trifluoromethylbenzyl, 3,5-dimethoxybenzyl or 2,2-diphenylethyl group,
$R_3$ denotes a $C_{2-4}$-alkyl group, an alkoxy, or alkylthio group each having 2 or 3 carbon atoms in the alkyl moiety, is cyclopropyl or cyclobutyl group and
$R_4$ denotes a group which may be converted into a carboxy group in vivo, or a carboxy or 1H-tetrazolyl group,
and the salts thereof.

**4.** Benzimidazoles of general formula I according to claim 1, wherein
$R_1$, $R_2$ and $R_3$ are defined as in claims 1 to 3 and
$R_4$ denotes a carboxy group or a group of formulae

- CO - OR',

- CO - O - (HCR'') - O - CO - R''' and

- CO - O - (HCR'') - O - CO - OR''',

wherein
R' denotes a straight chained or branched $C_{1-6}$-alkyl group, a $C_{5-7}$-cycloalkyl group, a benzyl, 1-phenylethyl, 2-phenylethyl, 3-phenylpropyl, methoxymethyl or cinnamyl group,
R'' denotes a hydrogen atom or a methyl group and
R''' denotes a straight-chained or branched $C_{1-6}$-alkyl group, a $C_{5-7}$-cycloalkyl group, a phenyl, benzyl, 1-phenylethyl, 2-phenylethyl or 3-phenylpropyl group,
and the salts thereof.

**5.** Benzimidazole of general formula I according to claim 1, wherein
$R_1$ denotes a hydrogen atom or a methyl group,
$R_2$ in the 6-position denotes an oxazol-4-yl or thiazol-4-yl group substituted in the 2-position by a phenyl group or an imidazol-4-yl group optionally substituted in the 2-position by a methyl group, which is substituted in the 1-position by a $C_{1-7}$-alkyl group, which is substituted in the 1-, 2-, 3-, 4-, 5-, 6- or 7-position by a carboxy, methoxycarbonyl, dimethylaminocarbonyl or morpholinocarbonyl group, by a $C_{2-4}$-alkyl group which is substituted in the 2-, 3- or 4-position by a hydroxy, methoxy, 2-methoxyethoxy, dimethylamino, diethylamino, pyrrolidino, piperidino, morpholino or imidazol-1-yl group, by a 2,2,2-trifluoroethyl, 3,3,3-trifluoropropyl, cyclopropylmethyl, cyclobutylmethyl, cyclopentylmethyl, cyclohexylmethyl or 4-fluorobenzyl group,
$R_3$ denotes a $C_{2-4}$-alkyl group, an ethoxy, ethylthio, cyclopropyl or cyclobutyl group and
$R_4$ denotes a carboxy or 1H-tetrazolyl group,
and the salts thereof.

**6.** The following compounds of general formula I according to claim 1:

(i) 4'-[(2-n-propyl-4-methyl-6-(1-(2-N-morpholinoethyl)-imidazol-4-yl)-benzimidazol-1-yl)-methyl]-biphenyl-2-carboxylic acid,

(ii) 4'-[(2-n-propyl-4-methyl-6-(1-(2-methoxyethoxy-2-ethyl)-imidazol-4-yl)-benzimidazol-1-yl)-methyl]-biphenyl-2-carboxylic acid,

(iii) 4'-[(2-ethyl-4-methyl-6-(1-(2-diethylaminoethyl)-imidazol-4-yl)-benzimidazol-1-yl)-methyl]-2-(1H-tetrazol-5-yl)-biphenyl,

(iv) 4'-[(2-ethyl-4-methyl-6-(1-(3-N-piperidinopropyl)-imidazol-4-yl)-benzimidazol-1-yl)-methyl]-2-(1H-tetrazol-5-yl)-biphenyl,

(v) 4'-[(2-n-propyl-4-methyl-6-(1-(3-dimethylaminopropyl)-imidazol-4-yl)-benzimidazol-1-yl)- methyl]-biphenyl-2-carboxylic acid,

(vi) 4'-[(2-ethyl-4-methyl-6-(1-(2-N-morpholinoethyl)-imidazol-4-yl)-benzimidazol-1-yl)-methyl]-biphenyl-2-carboxylic acid,

(vii) 4'-[(2-ethyl-4-methyl-6-(1-(2-N-morpholinoethyl)-imidazol-4-yl)-benzimidazol-1-yl)-methyl]-2-(1H-tetrazol-5-yl)-biphenyl,

(viii) 4'-[(2-ethyl-4-methyl-6-(1-(2-N-pyrrolidinoethyl)-imidazol-4-yl)-benzimidazol-1-yl)-methyl]-biphenyl-2-carboxylic acid,

(ix) 4'-[(2-ethyl-4-methyl-6-(1-(2-N-pyrrolidinoethyl)-imidazol-4-yl)-benzimidazol-1-yl)-methyl]-2-(1H-tetrazol-5-yl)-biphenyl,

(x) 4'-[(2-ethyl-4-methyl-6-(1-(2-diethylaminoethyl)-imidazol-4-yl)-benzimidazol-1-yl)-methyl]-biphenyl-2-carboxylic acid,

(xi) 4'-[(2-ethyl-4-methyl-6-(1-(3-N-piperidinopropyl)-imidazol-4-yl)-benzimidazol-1-yl)-methyl]-biphenyl-2-carboxylic acid

and the salts thereof.

**7.** 4'-[(2-n-Propyl-4-methyl-6-(1-(2-N-morpholinoethyl)-imidazol-4-yl)-benzimidazol-1-yl)-methyl]-biphenyl-2-carboxylic acid and the salts thereof.

**8.** Physiologically acceptable addition salts of the compounds according to at least one of claims 1 to 7 with inorganic or organic acids or bases.

**9.** Pharmaceutical compositions containing a compound according to at least one of claims 1 to 7 or a physiologically acceptable addition salt according to claim 8, optionally together with one or more inert carriers and/or diluents.

**10.** Use of a compound according to at least one of claims 1 to 8 for preparing a pharmaceutical composition with an angiotensin-antagonistic activity.

**11.** Process for preparing a pharmaceutical composition according to claim 9, characterised in that a compound according to at least one of claims 1 to 8 is incorporated in one or more inert carriers and/or diluents by a non-chemical method.

**12.** Process for preparing the benzimidazoles according to claims 1 to 8, characterised in that

a) a compound of general formula

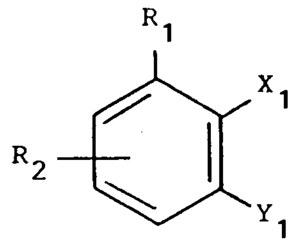

(II)

optionally formed in the reaction mixture, wherein $R_1$ and $R_2$ are defined as in claims 1 to 7, one of the groups $X_1$ or $Y_1$ represents a group of general formula

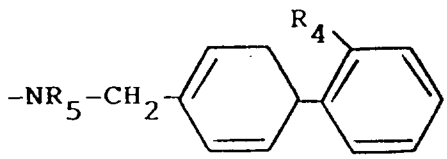

and the other group $X_1$ or $Y_1$ represents a group of the general formula

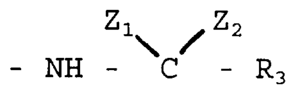

wherein
$R_3$ and $R_4$ are defined as in claims 1 to 7,
$R_5$ represents a hydrogen atom or an $R_3CO$ group, wherein $R_3$ is defined as hereinbefore,
$Z_1$ and $Z_2$, which may be identical or different, represent optionally substituted amino groups or hydroxy or mercapto groups optionally substituted by $C_{1-6}$-alkyl groups or
$Z_1$ and $Z_2$ together represent an oxygen or sulphur atom, an imino group optionally substituted by a $C_{1-3}$-alkyl group, or a alkylenedioxy or alkylenedithio group each having 2 or 3 carbon atoms, is cyclised, and an N-oxide optionally obtained in this way is reduced, or

b) a benzimidazole of general formula

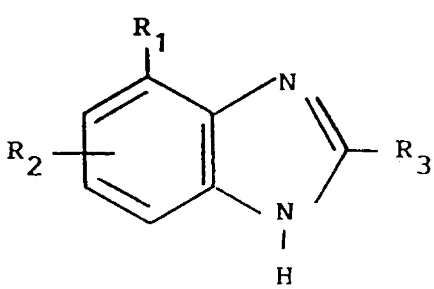

(III)

wherein
$R_1$ to $R_3$ are defined as in claims 1 to 7, is reacted with a biphenyl compound of general formula

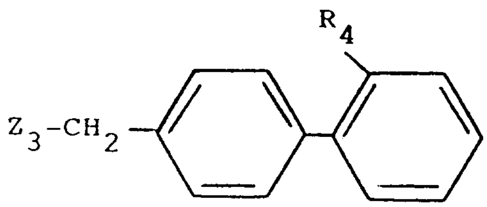

(IV)

wherein
$R_4$ is defined as in claims 1 to 7 and
$Z_3$ represents a nucleophilic leaving group, or

c) in order to prepare a compound of general formula I wherein $R_4$ represents a carboxy group, a compound of general formula

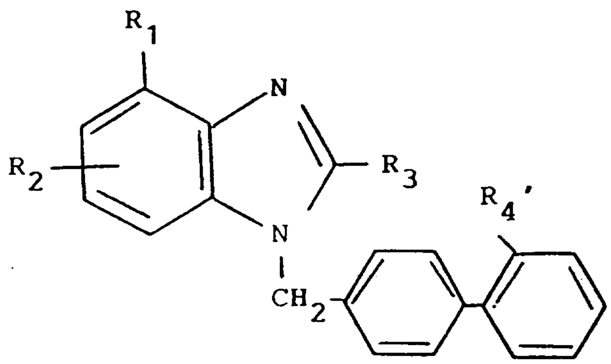

(V)

wherein
$R_1$ to $R_3$ are defined as in claims 1 to 7 and
$R_4$' represents a group which may be converted into a carboxy group by hydrolysis, thermolysis or hydrogenolysis, is converted into a corresponding carboxy compound, or

d) in order to prepare a compound of general formula I wherein $R_4$ represents a 1H-tetrazolyl group, a protecting group is cleaved from a compound of general formula

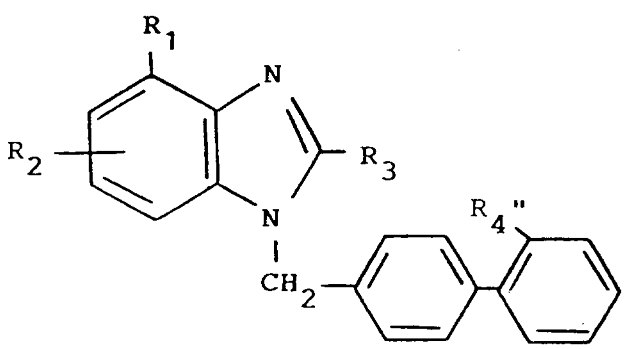

(VI)

wherein
$R_1$, $R_2$ and $R_3$ are defined as in claims 1 to 7 and
$R_4$" represents a 1H-tetrazolyl group protected in the 1- or 3-position by a protecting group, or

e) in order to prepare a compound of general formula I wherein $R_4$ represents a 1H-tetrazolyl group, a compound of general formula

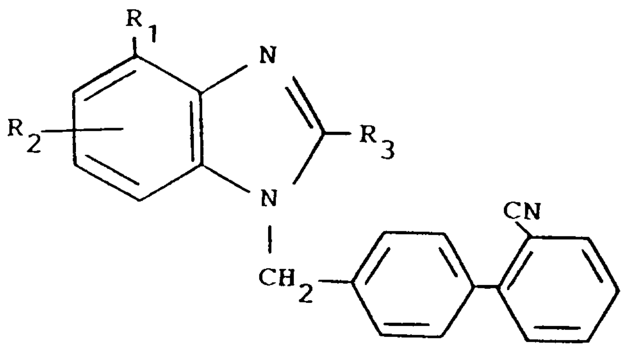

(VII)

wherein
$R_1$ to $R_3$ are defined as in claims 1 to 7, is reacted with hydrazoic acid or the salts thereof, and
subsequently, if desired, a compound of general formula I thus obtained wherein $R_4$ denotes a carboxy group is converted by esterification into a corresponding compound of general formula I wherein $R_4$ denotes a group which may be converted in vivo into a carboxy group, and/or
a compound of general formula I thus obtained wherein $R_2$ denotes an imidazol-4-yl group substituted in the 1-position by a $C_{2-4}$-alkyl group, wherein the alkyl group is substituted in 2-, 3- or 4-position by an alkoxy or alkoxyalkoxy group, is converted by ether splitting into a corresponding compound of general formula I wherein $R_2$ denotes an imidazol-4-yl group substituted in the 1-position by a $C_{2-4}$-alkyl group, the alkyl group being substituted in the 2-, 3- or 4-position by a hydroxy group, and
if necessary a protecting group used during reactions a) to e) in order to protect any reactive groups is cleaved and/or
subsequently, if desired, the 1-isomer is separated from a 1-, 3-isomer mixture of a compound of general formula I thus obtained by isomer separation or
a compound of general formula I thus obtained is converted into an addition salt thereof, more particularly, for pharmaceutical use, into a physiologically acceptable salt thereof with an inorganic or organic acid or base.

## Revendications

1. Benzimidazoles de formule générale

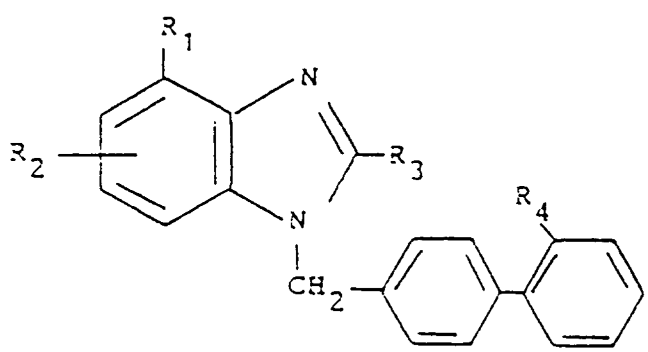

(I)

dans laquelle
$R_1$ représente un groupe alkyle de 1 à 3 atomes de carbone, un atome d'hydrogène, de fluor, de chlore ou de brome,
$R_2$ représente un groupe oxazol-4-yle ou thiazol-4-yle substitué en position 2 par un groupe phényle ou
un groupe imidazol-4-yle éventuellement substitué en position 2 par un groupe alkyle de 1 à 6 atomes de carbone ou par un groupe phényle, le groupe imidazol-4-yle étant substitué en position 1 par un groupe alkyle de 1 à 7 atomes de carbone qui est substitué en position 1, 2, 3, 4, 5, 6 ou 7 par un groupe carboxyle, alcoxycarbonyle, aminocarbonyle, alkylaminocarbonyle, dialkylaminocarbonyle, morpholinocarbonyle, thiomorpholinocarbonyle ou 1-oxydo-thiomorpholinocarbonyle, par un groupe alkyle de 2 à 4 atomes de carbone qui est substitué en position 2, 3 ou 4 par un groupe hydroxyle, alcoxy, alcoxyalcoxy, dialkylamino, pyrrolidino, pipéridino, hexaméthylénimino, morpholino, thiomorpholino, 1-oxydo-thiomorpholino ou imidazol-1-yle, par un groupe alkyle qui est substitué par un

groupe trifluorométhyle, par un groupe cycloalkyle de 3 à 7 atomes de carbone ou par un groupe phényle mono- ou disubstitué par des atomes de fluor ou de chlore, par des groupes trifluorométhyle, méthyle ou méthoxy, ou par un groupe alkyle substitué par deux groupes phényle, où, sauf indication contraire, les parties alkyle et alcoxy citées précédemment peuvent contenir à chaque fois 1 à 3 atomes de carbone,
$R_3$ représente un groupe alkyle de 2 à 4 atomes de carbone, un groupe alcoxy ou alkylthio de 2 ou 3 atomes de carbone dans la partie alkyle dans chaque cas, un groupe cyclopropyle ou cyclobutyle et
$R_4$ représente un groupe qui peut être converti in vivo en un groupe carboxyle, un groupe carboxyle, cyano, 1H-tétrazolyle, 1-triphénylméthyl-tétrazolyle ou 2-triphénylméthyl-tétrazolyle,
ainsi que les composés

a) acide 4'-[(2-n-propyl-4-méthyl-6-(1-(2-phényléthyl)-imidazol-4-yl)-benzimidazol-1-yl)-méthyl]-biphényl-2-carboxylique,
b) acide 4'-[(2-n-propyl-4-méthyl-6-(2-méthyl-oxazol-4-yl)-benzimidazol-1-yl)-méthyl]-biphényl-2-carboxylique,
c) 4'-[(2-n-propyl-4-méthyl-6-(2-méthyl-oxazol-4-yl)-benzimidazol-1-yl)-méthyl]-2-(1H-tétrazol-5-yl)-biphényle,
d) 4'-[(2-éthoxy-6-(1-isopropyl-imidazol-4-yl)-benzimidazol-1-yl)-méthyl]-2-(1H-tétrazol-5-yl)-biphényle,
e) acide 4'-[(2-éthoxy-6-(1-isopropyl-imidazol-4-yl)-benzimidazol-1-yl)-méthyl]-biphényl-2-carboxylique,
f) 4'-[(2-éthoxy-5-(1-isopropyl-imidazol-4-yl)-benzimidazol-1-yl)-méthyl]-2-(1H-tétrazol-5-yl)-biphényle,
g) acide 4'-[(2-n-propyl-4-méthyl-6-(1-cycloheptyl-imidazol-4-yl)-benzimidazol-1-yl)-méthyl]-biphényl-2-carboxylique,
h) 4'-[(2-n-propyl-4-méthyl-6-(1-cycloheptyl-imidazol-4-yl)-benzimidazol-1-yl)-méthyl]-2-(1H-tétrazol-5-yl)-biphényle,
i) 4'-[(2-n-propyl-4-méthyl-6-(1-(1-n-propyl-n-butyl)-imidazol-4-yl)-benzimidazol-1-yl)-méthyl]-2-(1H-tétrazol-5-yl)-biphényle,
j) acide 4'-[(2-n-propyl-4-méthyl-6-(1,2-diméthyl-imidazol-4-yl)-benzimidazol-1-yl)-méthyl]-biphényl-2-carboxylique,
k) 4'-[(2-n-propyl-4-méthyl-6-(1,2-diméthyl-imidazol-4-yl)-benzimidazol-1-yl)-méthyl]-2-(1H-tétrazol-5-yl)-biphényle,
l) acide 4'-[(2-n-propyl-4-méthyl-6-(2-méthyl-thiazol-4-yl)-benzimidazol-1-yl)-méthyl]-biphényl-2-carboxylique et
k) 4'-[(2-n-propyl-4-méthyl-6-(2-méthyl-thiazol-4-yl)-benzimidazol-1-yl)-méthyl]-2-(1H-tétrazol-5-yl)-biphényle,
et leurs sels.

**2.** Benzimidazoles de formule générale I selon la revendication 1, dans lesquels
$R_1$ représente un groupe alkyle de 1 à 3 atomes de carbone, un atome d'hydrogène, de fluor, de chlore ou de brome,
$R_2$ en position 6 représente un groupe oxazol-4-yle ou thiazol-4-yle substitué en position 2 par un groupe phényle ou
un groupe imidazol-4-yle éventuellement substitué en position 2 par un groupe alkyle de 1 à 6 atomes de carbone ou par un groupe phényle, le groupe imidazol-4-yle étant substitué en position 1 par un groupe alkyle de 1 à 7 atomes de carbone qui est substitué en position 1, 2, 3, 4, 5, 6 ou 7 par un groupe carboxyle, alcoxycarbonyle, aminocarbonyle, alkylaminocarbonyle, dialkylaminocarbonyle, morpholinocarbonyle, thiomorpholinocarbonyle ou 1-oxydo-thiomorpholinocarbonyle, par un groupe alkyle de 2 à 4 atomes de carbone qui est substitué en position 2, 3 ou 4 par un groupe hydroxyle, alcoxy, alcoxyalcoxy, dialkylamino, pyrrolidino, pipéridino, hexaméthylénimino, morpholino, thiomorpholino, 1-oxydo-thiomorpholino ou imidazol-1-yle, par un groupe alkyle qui est substitué par un groupe trifluorométhyle, par un groupe cycloalkyle de 3 à 7 atomes de carbone ou par un groupe phényle mono- ou disubstitué par des atomes de fluor ou de chlore, par des groupes trifluorométhyle, méthyle ou méthoxy, ou par un groupe alkyle substitué par deux groupes phényle, où, sauf indication contraire, les parties alkyle et alcoxy citées précédemment peuvent contenir à chaque fois 1 à 3 atomes de carbone,
$R_3$ représente un groupe alkyle de 2 à 4 atomes de carbone, un groupe alcoxy ou alkylthio de 2 ou 3 atomes de carbone dans la partie alkyle dans chaque cas, un groupe cyclopropyle ou cyclobutyle et
$R_4$ représente un groupe qui peut être converti in vivo en un groupe carboxyle, un groupe carboxyle, cyano, 1H-tétrazolyle, 1-triphénylméthyl-tétrazolyle ou 2-triphénylméthyl-tétrazolyle,
et leurs sels.

**3.** Benzimidazoles de formule générale I selon la revendication 1, dans laquelle
$R_1$ représente un atome d'hydrogène ou de chlore ou un groupe méthyle,
$R_2$ en position 6 représente un groupe oxazol-4-yle ou thiazol-4-yle substitué en position 2 par un groupe phényle ou
un groupe imidazol-4-yle éventuellement substitué en position 2 par un groupe méthyle, qui est substitué en position 1 par un groupe alkyle de 1 à 7 atomes de carbone qui est substitué en position 1, 2, 3, 4, 5, 6 ou 7 par un groupe carboxyle, méthoxycarbonyle, aminocarbonyle, méthylaminocarbonyle, éthylaminocarbonyle, n-propylaminocarbonyle, diméthylaminocarbonyle, morpholinocarbonyle, thiomorpholinocarbonyle ou 1-oxydo-thiomorpholinocarbonyle, par un groupe alkyle de 2 à 4 atomes de carbone qui est substitué en position 2, 3 ou 4 par un groupe hydroxyle, méthoxy, 2-méthoxyéthoxy, diméthylamino, diéthylamino, pyrrolidino, pipéridino, morpholino ou imidazol-1-yle, par un groupe 2,2,2-trifluoroéthyle, 3,3,3-trifluoropropyle, cyclopropylméthyle, cyclobutylméthyle, cyclopentylméthyle, cyclohexylméthyle, 4-fluorobenzyle, 3-chlorobenzyle, 4-méthylbenzyle, 4-trifluorométhylbenzyle, 3,5-diméthoxybenzyle ou 2,2-diphényléthyle,
$R_3$ représente un groupe alkyle de 2 à 4 atomes de carbone, un groupe alcoxy ou alkylthio de 2 ou 3 atomes de carbone dans la partie alkyle dans chaque cas, un groupe cyclopropyle ou cyclobutyle et
$R_4$ représente un groupe qui peut être converti in vivo en un groupe carboxyle, un groupe carboxyle ou 1H-tétrazolyle,
et leurs sels.

**4.** Benzimidazoles de formule générale I selon la revendication 1, dans laquelle
$R_1$, $R_2$ et $R_3$ sont définis comme dans les revendications 1 à 3 et
$R_4$ représente un groupe carboxyle ou un groupe selon les formules

- CO - OR',

- CO - O - (HCR'') - O - CO - R''' et

- CO - O - (HCR'') - O - CO - OR'''

dans lesquelles
R' représente un groupe alkyle linéaire ou ramifié de 1 à 6 atomes de carbone, un groupe cycloalkyle de 5 à 7 atomes de carbone, un groupe benzyle, 1-phényléthyle, 2-phényléthyle, 3-phénylpropyle, méthoxyméthyle ou cinnamyle,
R'' représente un atome d'hydrogène ou un groupe méthyle et
R''' représente un groupe alkyle linéaire ou ramifié de 1 à 6 atomes de carbone, un groupe cycloalkyle de 5 à 7 atomes de carbone, un groupe phényle, benzyle, 1-phényléthyle, 2-phényléthyle ou 3-phénylpropyle
et leurs sels.

**5.** Benzimidazoles de formule générale I selon la revendication 1, dans laquelle
$R_1$ représente un atome d'hydrogène ou un groupe méthyle,
$R_2$ en position 6 représente un groupe oxazol-4-yle ou thiazol-4-yle substitué en position 2 par un groupe phényle ou
un groupe imidazol-4-yle éventuellement substitué en position 2 par un groupe méthyle, qui est substitué en position 1 par un groupe alkyle de 1 à 7 atomes de carbone qui est substitué en position 1, 2, 3, 4, 5, 6 ou 7 par un groupe carboxyle, méthoxycarbonyle, diméthylaminocarbonyle ou morpholinocarbonyle, par un groupe alkyle de 2 à 4 atomes de carbone qui est substitué en position 2, 3 ou 4 par un groupe hydroxyle, méthoxy, 2-méthoxyéthoxy, diméthylamino, diéthylamino, pyrrolidino, pipéridino, morpholino ou imidazol-1-yle, par un groupe 2,2,2-trifluoroéthyle, 3,3,3-trifluoropropyle, cyclopropylméthyle, cyclobutylméthyle, cyclopentylméthyle, cyclohexylméthyle ou 4-fluorobenzyle, $R_3$ représente un groupe alkyle de 2 à 4 atomes de carbone, un groupe éthoxy, éthylthio, cyclopropyle ou cyclobutyle et $R_4$ représente un groupe carboxyle ou 1H-tétrazolyle,
et leurs sels.

**6.** Composés suivants de formule générale I selon la revendication 1:

(i) acide 4'-[(2-n-propyl-4-méthyl-6-(1-(2-N-morpholinoéthyl)-imidazol-4-yl)-benzimidazol-1-yl)-méthyl]-biphényl-2-carboxylique,
(ii) acide 4'-[(2-n-propyl-4-méthyl-6-(1-(2-méthoxyéthoxy-2-éthyl)-imidazol-4-yl)-benzimidazol-1-yl)-méthyl]-biphényl-2-carboxylique,
(iii) 4'-[(2-éthyl-4-méthyl-6-(1-(2-diéthylaminoéthyl)-imidazol-4-yl)-benzimidazol-1-yl)-méthyl]-2-(1H-tétrazol-5-yl)-biphényle,
(iv) 4'-[(2-éthyl-4-méthyl-6-(1-(3-N-pipéridinopropyl)-imidazol-4-yl)-benzimidazol-1-yl)-méthyl]-2-(1H-tétrazol-5-yl)-biphényle,
(v) acide 4'-[(2-n-propyl-4-méthyl-6-(1-(3-diméthylaminopropyl)-imidazol-4-yl)-benzimidazol-1-yl)-méthyl]-biphényl-2-carboxylique,

(vi) acide 4'-[(2-éthyl-4-méthyl-6-(1-(2-N-morpholinoéthyl)-imidazol-4-yl)-benzimidazol-1-yl)-méthyl]-biphényl-2-carboxylique,
(vii) 4'-[(2-éthyl-4-méthyl-6-(1-(2-N-morpholinoéthyl)-imidazol-4-yl)-benzimidazol-1-yl)-méthyl]-2-(1H-tétrazol-5-yl)-biphényle,
(viii) acide 4'-[(2-éthyl-4-méthyl-6-(1-(2-N-pyrrolidinoéthyl)-imidazol-4-yl)-benzimidazol-1-yl)-méthyl]-biphényl-2-carboxylique,
(ix) 4'-[(2-éthyl-4-méthyl-6-(1-(2-N-pyrrolidinoéthyl)-imidazol-4-yl)-benzimidazol-1-yl)-méthyl]-2-(1H-tétrazol-5-yl)-biphényle,
(x) acide 4'-[(2-éthyl-4-méthyl-6-(1-(2-diéthylaminoéthyl)-imidazol-4-yl)-benzimidazol-1-yl)-méthyl]-biphényl-2-carboxylique,
(xi) acide 4'-[(2-éthyl-4-méthyl-6-(1-(3-N-pipéridinopropyl)-imidazol-4-yl)-benzimidazol-1-yl)-méthyl]-biphényl-2-carboxylique,

et leurs sels.

7. Acide 4'-[(2-n-propyl-4-méthyl-6-(1-(2-N-morpholinoéthyl)-imidazol-4-yl)-benzimidazol-1-yl)-méthyl]-biphényl-2-carboxylique et ses sels.

8. Sels physiologiquement acceptables des composés selon au moins l'une des revendications 1 à 7 avec des acides ou bases inorganiques ou organiques.

9. Médicament contenant un composé selon au moins l'une des revendications 1 à 7 ou un sel physiologiquement acceptable selon la revendication 8 et éventuellement un ou plusieurs supports et/ou diluants inertes.

10. Utilisation d'un composé selon au moins l'une des revendications 1 à 8 pour la préparation d'un médicament à effet antagoniste de l'angiotensine.

11. Procédé de préparation d'un médicament selon la revendication 9 caractérisé en ce qu'un composé selon au moins l'une des revendications 1 à 8 est incorporé par voie non chimique dans un ou plusieurs supports et/ou diluants inertes.

12. Procédé de préparation des benzimidazoles selon les revendications 1 à 8 caractérisé en ce que

a) un composé éventuellement formé dans le mélange réactionnel, de formule générale

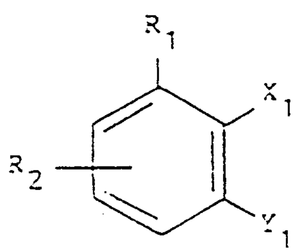

(II)

dans laquelle
$R_1$ et $R_2$ sont définis comme dans les revendications 1 à 7, l'un des restes $X_1$ et $Y_1$ représente un groupe de formule générale

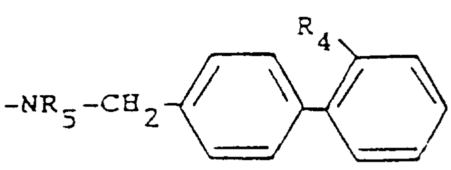

et l'autre des restes $X_1$ et $Y_1$ représente un groupe de formule générale

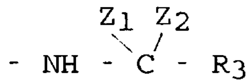

où

$R_3$ et $R_4$ sont définis comme dans les revendications 1 à 7,

$R_5$ représente un atome d'hydrogène ou un groupe $R_3CO$, $R_3$ étant défini comme indiqué précédemment,

$Z_1$ et $Z_2$, qui peuvent être identiques ou différents, représentent des groupes amino éventuellement substitués ou des groupes hydroxyle ou mercapto éventuellement substitués par des groupes alkyle de 1 à 6 atomes de carbone ou

$Z_1$ et $Z_2$ représentent ensemble un atome d'oxygène ou de soufre, un groupe imino éventuellement substitué par un groupe alkyle de 1 à 3 atomes de carbone, un groupe alkylènedioxy ou alkylènedithio de 2 ou 3 atomes de carbone dans chaque cas, est cyclisé et un N-oxyde éventuellement ainsi obtenu est réduit ou

b) un benzimidazole de formule générale

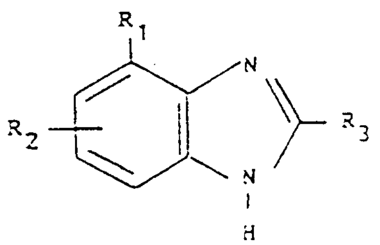

(III)

dans laquelle

$R_1$ à $R_3$ sont définis comme dans les revendications 1 à 7, est mis à réagir avec un composé biphénylique de formule générale

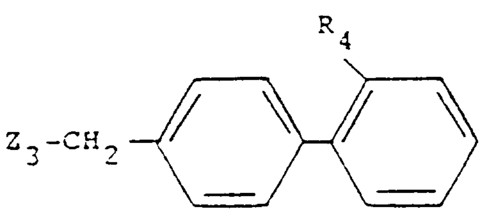

(IV)

dans laquelle

$R_4$ est défini comme dans les revendications 1 à 7 et

$Z_3$ représente un groupe partant nucléophile, ou

c) pour la préparation d'un composé de formule générale I dans laquelle $R_4$ représente un groupe carboxyle, un composé de formule générale

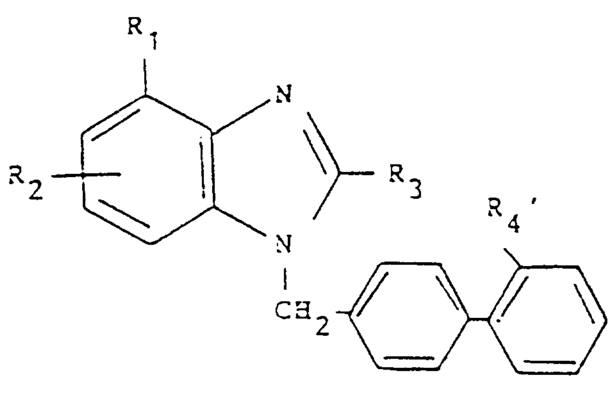

(V)

dans laquelle
$R_1$ à $R_3$ sont définis comme dans les revendications 1 à 7 et $R_4'$ représente un groupe qui peut être converti en un groupe carboxyle par hydrolyse, thermolyse ou hydrogénolyse, est converti en un composé carboxylé correspondant ou

d) pour la préparation d'un composé de formule générale I dans laquelle $R_4$ représente un groupe 1H-tétrazolyle, un reste protecteur est clivé d'un composé de formule générale

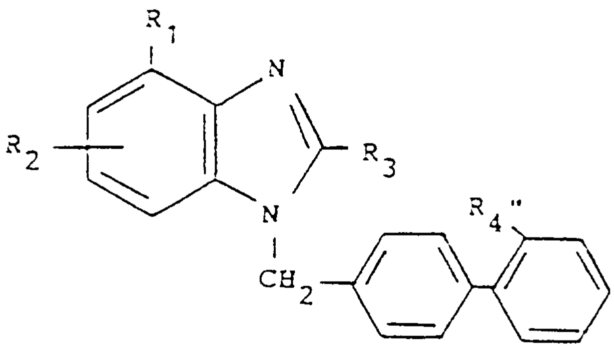

(VI)

dans laquelle
$R_1$, $R_2$ et $R_3$ sont définis comme dans les revendications 1 à 7 et
$R_4''$ représente un groupe 1H-tétrazolyle protégé en position 1 ou 3 par un reste protecteur, ou

e) pour la préparation d'un composé de formule générale I dans laquelle $R_4$ représente un groupe 1H-tétrazolyle, un composé de formule générale

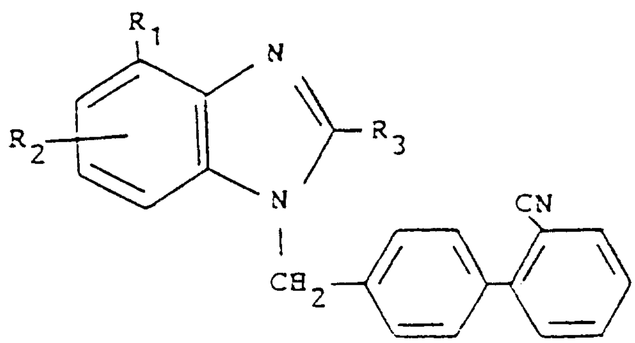

(VII)

dans laquelle
$R_1$ à $R_3$ sont définis comme dans les revendications 1 à 7, est mis à réagir avec l'acide azothydrique ou ses sels puis,
si on le souhaite, un composé ainsi obtenu de formule générale I dans laquelle $R_4$ représente un groupe carboxyle, est converti par estérification en un composé correspondant de formule générale I dans laquelle $R_4$ représente un groupe qui peut être converti in vivo en un groupe carboxyle et/ou un composé ainsi obtenu de formule générale I dans laquelle $R_2$ représente un groupe imidazol-4-yle qui est substitué en position 1 par un groupe alkyle de 2 à 4 atomes de carbone, le groupe alkyle étant substitué en position 2, 3 ou 4 par un groupe alcoxy ou alcoxyalcoxy, est converti par clivage d'éther en un composé correspondant de formule générale I dans laquelle $R_2$ représente un groupe imidazol-4-yle qui est substitué en position 1 par un groupe alkyle de 2 à 4 atomes de carbone, le groupe alkyle étant substitué en position 2, 3 ou 4 par un groupe hydroxyle et
si nécessaire un reste protecteur utilisé pendant les réactions a) à e) pour la protection de groupes réactifs est clivé et/ou
l'isomère 1 est ensuite séparé par séparation d'isomères d'un mélange ainsi obtenu d'isomères 1, 3 d'un composé de formule générale I ou
un composé de formule générale I ainsi obtenu est converti en son sel, en particulier pour l'utilisation pharmaceutique en son sel physiologiquement acceptable avec un acide ou une base inorganique ou organique.